# EUROPEAN PATENT APPLICATION

(11) **EP 4 450 614 A1**
(43) Date of publication of application: **23.10.2024**
(21) Application number: 23168792.2
(22) Date of filing: 19.04.2023
(51) Int. Cl.: C12N 5/0783, C12N 5/10, A61K 35/17, A61K 39/00, A61P 35/02

(54) **TREATMENT OF LEUKEMIA BY COMBINING A HYPOMETHYLATING AGENT IN COMBINATION WITH ENGINEERED CAR-NK CELLS**

(71) Applicant: Johann-Wolfgang-Goethe-Universität Frankfurt am Main, 60323 Frankfurt am Main (DE); Albert-Ludwigs-Universität Freiburg, 79098 Freiburg (DE)
(72) Inventor: Ullrich, Evelyn, 60596 Frankfurt am Main (DE); Bexte, Tobias, 60596 Frankfurt am Main (DE); Wendel, Philipp, 60596 Frankfurt am Main (DE); Zeiser, Robert, 79106 Freiburg (DE)
(74) Representative: Krauss, Jan

(57) **Abstract**

The present invention relates to non-virus-based method for producing a CAR-NK cell expressing an antigen-targeting chimeric antigen receptor (CAR) and a recombinant CAR-NK cell as produced, in particular a CAR-NK cell targeting the cancer-associated antigen CLEC12A, wherein optionally at least one immune checkpoint receptor is inactivated in the CAR-NK cell. Furthermore, the present invention relates to medical uses of the CAR-NK cell for the prevention or treatment of leukemia, such as AML, comprising a combination treatment with a hypomethylating agent, such as azacitidine (AZA) or decitabine. The present invention further relates to a CAR-construct, comprising a modified CD8α or CD28 transmembrane domain. Furthermore, highly functional immune checkpoint inactivated CAR-NK cells that target cancer-associated antigens or are adapted for a treatment of autoimmune diseases are provided.

## Description

The present invention relates to non-virus-based method for producing a CAR-NK cell expressing an antigen-targeting chimeric antigen receptor (CAR) and a recombinant CAR-NK cell as produced, in particular a CAR-NK cell targeting the cancer-associated antigen CLEC12A, wherein optionally at least one immune checkpoint receptor is inactivated in the CAR-NK cell. Furthermore, the present invention relates to medical uses of the CAR-NK cell for the prevention or treatment of leukemia, such as AML, comprising a combination treatment with a hypomethylating agent, such as azacitidine (AZA) or decitabine. The present invention further relates to a CAR-construct, comprising a modified CD8α or CD28 transmembrane domain. Furthermore, highly functional immune checkpoint inactivated CAR-NK cells that target cancer-associated antigens or are adapted for a treatment of autoimmune diseases are provided.

### Background of the invention

Acute myeloid leukemia (AML) is the most common form of leukemia in adults with a high morbidity and mortality rate. Especially patients who are not eligible for allo-HCT are prone to therapy-resistance and early relapse. Therefore, the development of new treatment concepts, in particular immunotherapy approaches with minimal side effects, is of urgent medical need.

The prognosis of high-risk AML remains unfavorable for the majority of patients who are not eligible for allogeneic hematopoietic cell transplantation (allo-HCT). For these patients, treatment is currently palliative. Also, for younger patients eligible for standard induction chemotherapy and allo-HCT, a considerable number of patients eventually relapses and succumbs to the disease.

An important immunotherapy-based treatment approach with lower toxicity compared to allo-HCT are chimeric antigen receptor (CAR-) T or NK cells that could be administered to older AML patients unfit for allo-HCT or patients relapsing after allo-HCT.

While multiple CAR-T cell products targeting the CD 19 antigen on B cells were approved by the European Medicines Agency (EMA) and the American Food and Drug Administration (FDA) for several B-lymphoid malignancies based on the high and durable clinical responses, there is currently no CAR-T cell product approved for AML. The main reason is that current CAR-T cell products against AML also target normal myeloid progenitor cells that express the same antigen. Multiple antigens have been explored for CARs against AML cells, including CD123, CD33, FLT3, CD96, CD135, TIM3, CD244 and CD7.

An antigen that was frequently tested in clinical trials is CD123, the α-chain of the interleukin-3 receptor. Several pre-clinical and clinical studies have reported potent anti-leukemic activity with CD123-specific CAR-T cells against AML. However, myelotoxicity or reduced CAR-T cell efficacy due to the immunosuppressive environment induced by AML cells hindered its clinical approval.

In contrast to other target antigens expressed on the surface of AML blasts, the antigen C-type lectin domain family 12 member A (CLEC12A a.k.a. CLL-1) is highly expressed on AML cells and also on leukemia initiating cells or leukemic stem cells (LSC), but not expressed on healthy hematopoietic stem cells (HSCs). Therefore, different investigators have started to evaluate CLEC12A as a target for the treatment of AML using bispecific antibodies, CAR-T cells and antibody-drug conjugates (van Loo PF, et al. MCLA-117, a CLEC12AxCD3 bispecific antibody targeting a leukaemic stem cell antigen, induces T cell-mediated AML blast lysis. Expert Opin Biol Ther. 2019;7:721-733).

Nevertheless, immune escape of AML cells by downregulation of CLEC12A expression combined with the exhaustion of the applied CLEC12A-CAR-T cells promotes the selection of therapy resistant CLEC12A-negative AML cells.

In addition to cytotoxic T lymphocytes (CTLs), natural killer (NK) cells have recently become a novel tool for immunotherapy. Although these cell types utilize similar killing mechanisms for eliminating malignant or virally infected cells, their target recognition mechanism differs significantly. CTLs, as part of the adaptive immune response, recognize their targets via a wide variety of clonally rearranged T cell receptors (TCRs), while NK cells, as innate lymphoid cells, integrate activating and inhibitory signals received by their germline-encoded receptor repertoire. These distinct target recognition mechanisms allow complementary function and render NK cells as an attractive tool for immunotherapy.

Unfortunately, NK cells are typically more difficult to transfect than T cells, most likely due to enhanced resistance to the uptake of foreign nucleic acids as a first-line of defense against viral pathogens. Although the efficiency of lenti- and retroviral engineering of CAR NK cells has improved in recent years and led to promising results (Reindl LM, et al. Immunotherapy with NK cells: recent developments in gene modification open up new avenues. Oncoimmunology. 2020 Sep 2;9(1):1777651. doi: 10.1080/2162402X.2020.1777651. PMID: 33457093; PMCID: PMC7781759; Wendel P, et al. Arming Immune Cells for Battle: A Brief Journey through the Advancements of T and NK Cell Immunotherapy. Cancers (Basel). 2021 Mar 23;13(6):1481. doi: 10.3390/cancers13061481. PMID: 33807011; PMCID: PMC8004685), non-viral NK cell modification remains challenging, and has largely been limited to transient CAR expression (Schmidt P, Raftery MJ, Pecher G. Engineering NK Cells for CAR Therapy-Recent Advances in Gene Transfer Methodology. Front Immunol. 2021 Jan 7;11:611163. doi: 10.3389/fimmu.2020.611163. PMID: 33488617; PMCID: PMC7817882).

NK cells are defined as CD56⁺CD3⁻ lymphocytes and are traditionally classified into two main subsets based on the expression of CD56 and the low-affinity Fc gamma receptor 3A (FcyRIIIa, CD16). One population is primarily immunomodulatory with cells defined as CD56^{bright}CD16^{dim} while the other consists of CD56^{dim}CD16^{bright} NK cells displaying stronger cytotoxicity. Further functional subpopulations can be defined by the expression of several surface markers, e.g. chemokine receptors such as CXCR4, CX3CR1 or CCR7, which determine tissue distribution and homing capability as well as maturation markers such as CD27 or CD57, which define different developmental stages.

Allison M. et al. (in: Natural Killer Cell-Mediated Immunotherapy for Leukemia. Cancers (Basel). 2022 Feb 8;14(3):843. doi: 10.3390/cancers14030843. PMID: 35159109; PMCID: PMC8833963) review NK cells and NK cell receptors, functional impairment of NK cells in leukemia, NK cell immunotherapies currently under investigation including monoclonal antibodies (mAbs), adoptive transfer, chimeric antigen receptor-NKs (CAR-NKs), bi-specific/tri-specific killer engagers (BiKEs/TriKEs) and potential targets of NK cell-mediated immunotherapy for leukemia in the future.

Battin C, et al. (in: NKG2A-checkpoint inhibition and its blockade critically depends on peptides presented by its ligand HLA-E. Immunology. 2022 Aug;166(4):507-521. doi: 10.1111/imm.13515. Epub 2022 Jun 6. PMID: 35596615; PMCID: PMC9426624) describes checkpoint receptor receptor NKG2A, part of the NKG2x family of proteins, as a C-type lectin which dimerizes with CD94 (KLRD1). The formation of high-affinity HLA-E-peptide complexes could potentially contribute to resistance to ICI therapies targeting NKG2A.

Recent findings demonstrated promising combinatorial approaches of hypomethylating agents with CD123-targeting CAR-T cells (El Khawanky, N., Hughes, A., Yu, W. et al. Demethylating therapy increases anti-CD123 CAR T cell cytotoxicity against acute myeloid leukemia. Nat Commun 12, 6436 (2021). https://doi.org/10.1038/s41467-021-26683-0). However, on-target/off-tumor effects of CD123-targeting immune cells and immune suppression by components of the tumor microenvironment might complicate clinical translation. Furthermore, the use of CAR-T cells in clinical application is still mostly restricted to the generation of autologous cell products, which might fail in case of intensively pre-treated patients.

In view of the above, it is an object of the present invention to provide and to develop improved CAR-NK cell anti-cancer, in particular anti-leukemia, as well as anti-autoimmune disease therapies and strategies. Other objects and advantages of the present invention will become apparent to the person of skill when studying the following more detailed description of the present invention, including the Figures and examples.

According to a first aspect of the present invention the above object is solved by a method for producing a CAR-NK cell expressing an antigen-targeting chimeric antigen receptor (CAR) or a functional alternatively spliced transcript variant thereof, comprising the steps of a) providing primary NK cells, b) pretreating the primary NK cells for about 2 days in a suitable medium comprising about 10 ng/ml IL15, c) nucleofecting the cells with i) at least one Minicircle DNA encoding a transposon cassette element comprising a nucleic acid encoding the CAR or the functional alternatively spliced transcript variant thereof, and ii) a nucleic acid encoding at least one Sleeping Beauty (SB 100X) transposase using a suitable nucleofection program, such as, for example, DO100, and d) expanding the nucleofected cells in a suitable medium comprising about 10 ng/ml IL15.

The method according to the present invention provides a surprising transformation efficiency of the NK cells to at least 30%, preferably at least 40%, and more preferably to at least 50%, and a stable integration is achieved.

Preferred is a pharmaceutical composition, comprising the CAR-NK cell produced according to the present invention, further comprising a pharmaceutically acceptable diluent or carrier.

Further preferred is a method according to the present invention, wherein the CAR binds to an antigen selected from BCMA, CD19 and CLEC12A, and wherein the CAR is preferably genetically modified and optimized for stability (see below).

Further preferred is a method according to the present invention, further comprising the step of inactivating or rendering non-functional at least one immune checkpoint and/or regulatory receptor protein, such as, for example NKG2A, PD-1, and/or KIRS, wherein preferably the immune checkpoint receptor inactivation comprises a genetic deletion, a CRISPR/Cas introduced mutation, RNA interference (RNAi) and/or the recombinant expression of an immune checkpoint receptor inhibiting peptide.

According to a second aspect of the present invention the above object is solved by a recombinant CAR-NK cell, produced according to the method according to the present invention.

According to a third aspect of the present invention the above object is solved by a CAR-construct, comprising a modified CD8α transmembrane domain comprising between 3 to 5, preferably 4 additional amino acids at the N-terminus and between 1 to 3, preferably 2 additional amino acids at the C-terminus of the CD8α transmembrane (TM) domain.

According to a fourth aspect of the present invention the above object is solved by a nucleic acid encoding for the CAR-construct according to the present invention or an expression vector comprising said nucleic acid.

According to a fourth aspect of the present invention the above object is solved by a recombinant CAR-NK cell or CAR-T-cell, comprising the CAR-construct according to the present invention or expressing the nucleic acid or expression vector according to the present invention.

According to a fifth aspect of the present invention the above object is solved by a recombinant CAR-NK cell, expressing a CAR binding to the antigen CLEC12A or a functional alternatively spliced transcript variant thereof, wherein at least one immune checkpoint receptor protein, such as, for example NKG2A, PD-1, and/or KIRS, is inactivated, wherein preferably the immune checkpoint receptor inactivation comprises a genetic deletion, a CRISPR/Cas introduced mutation, RNA interference (RNAi) and/or the recombinant expression of an immune checkpoint receptor inhibiting peptide.

According to a sixth aspect of the present invention the above object is solved by a pharmaceutical composition, comprising the CAR-NK cell according to the present invention, the nucleic acid or the expression vector according to the present invention, or the CAR-NK cell or CAR-T-cell according to the present invention, together with at least one pharmaceutically acceptable diluent or carrier.

According to a seventh aspect of the present invention the above object is solved by a CAR-NK cell according to the present invention, the nucleic acid or the expression vector according to the present invention, the CAR-NK cell or CAR-T-cell according to the present invention or the pharmaceutical composition according to the present invention for use in medicine, in particular for use in the prevention or treatment of cancer, such as leukemia, or autoimmune diseases in a patient. Preferred is the use as above, wherein the prevention or treatment comprises a combination treatment with a hypomethylating agent, such as, for example, selected from azacitidine (AZA) or decitabine.

According to an eighth aspect of the present invention the above object is solved by a method for preventing or treating cancer, such as leukemia, or autoimmune diseases in a patient, comprising administering an effective amount of the CAR-NK cell according to the present invention, the nucleic acid or the expression vector according to the present invention, the CAR-NK cell or CAR-T-cell according to the present invention or the pharmaceutical composition according to the present invention to said patient.

Preferred is the method according to the present invention, wherein the prevention or treatment comprises a combination treatment with an effective amount of a hypomethylating agent, such as, for example, selected from azacitidine (AZA) or decitabine. The leukemia may be selected from the group consisting of leukemic cells expressing CLEC12A, acute myeloid leukemia (AML), and subtypes thereof.

As mentioned above, according to a first aspect thereof, the present invention provides a method for producing a CAR-NK cell expressing an antigen-targeting chimeric antigen receptor (CAR) or a functional alternatively spliced transcript variant thereof, comprising the steps of a) providing primary NK cells, b) pretreating the primary NK cells for about 2 days in a suitable medium comprising about 10 ng/ml IL15, c) nucleofecting the cells with i) at least one Minicircle DNA encoding a transposon cassette element comprising a nucleic acid encoding the CAR or the functional alternatively spliced transcript variant thereof, and ii) a nucleic acid encoding at least one Sleeping Beauty (SB100X) transposase using a suitable nucleofection program, such as, for example, DK100 or DK100, and d) expanding the nucleofected cells in a suitable medium comprising about 10 ng/ml IL15. Instead of IL15, an equivalent amount of a functionally related and preferably human active cytokine may be used, such as, for example, IL2. The cytokines may be used at a "low dose", i.e. at between 3 and 15 ng/ml, preferably at between 5 and 12 ng/ml, more preferably at about 10 ng/ml.

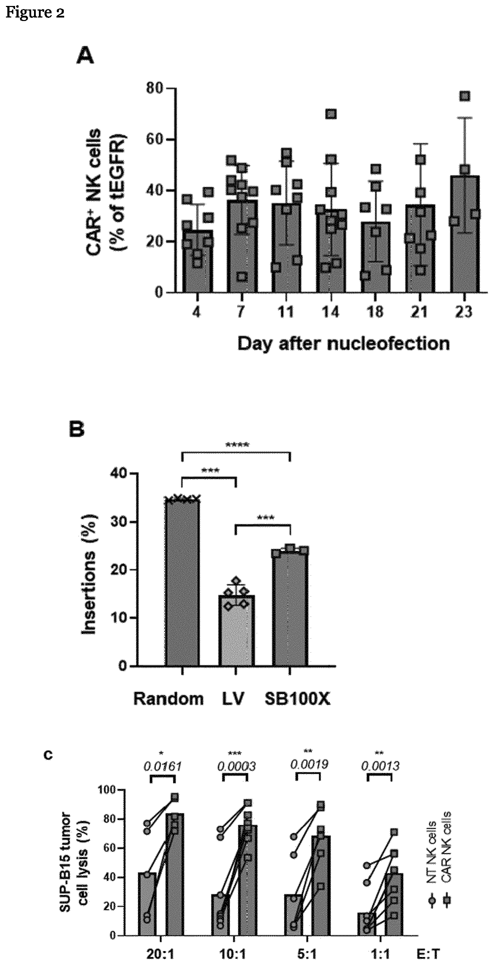

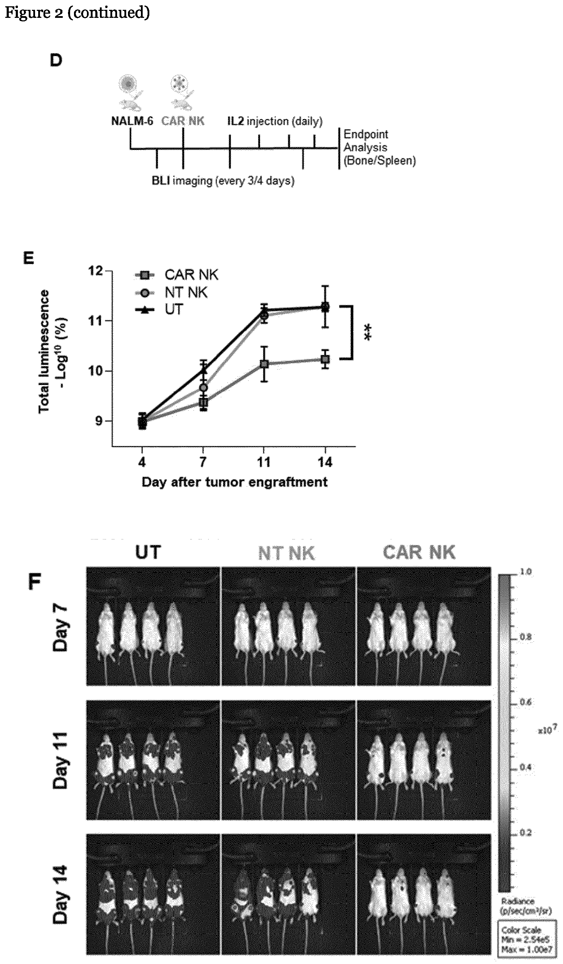

WO2017050884A1 discloses a method for the stable integration of transgenes into lymphocytes and other mammalian cells based on the use of an mRNA-encoded transposase (e.g. sleeping beauty transposase) in combination with a Minicircle DNA-encoded transposable element. The application also discloses to be able to modify natural killer cells. Nevertheless, it was found in the context of the experiments of the present invention that the method can not be used to efficiently transfect NK cells, and less than 10% of NK cells were shown to express CD19-CAR as used (see Monjezi et al., Leukemia, 2018, Figure 21). In contrast, the present invention provides a protocol that leads to more than about 40% of cells showing stable CAR-expression (see Figure 2).

Minicircle (MC) DNA vectors in combination with a transposon have been reported to lead to higher transposition rates compared with plasmids in hematopoietic and immune cells such as human CD34⁺ cells and T lymphocytes. In the context of the present invention, the inventors developed an optimized NK cell nucleofection protocol that enables the SB transposase-mediated mobilization of CAR transposons from MC DNA vectors, resulting in stable integration into the genome of NK cells. The data demonstrated that SB transposition results in long-lasting CAR expression with high viability of the effector cells after nucleofection. Importantly, the inventors observed a safer integration profile for SB 100X-based CAR transposition when compared to lentivirally transduced CAR-NK cells.

Bexte T., et al. (in: Non-Viral Sleeping Beauty Transposon Engineered CD19-CAR-NK Cells Show a Safe Genomic Integration Profile and High Antileukemic Efficiency. Blood (2021) 138 (Supplement 1): 2797) disclose that primary NK cells can be engineered using the non-viral *Sleeping Beauty* (SB) transposon/transposase system to stably express a CD19-CAR with a safe genomic integration profile and high anti-leukemic efficiency *in vitro* and *in vivo.* Primary NK cells were isolated from PBMCs from healthy donors. SB transposons vectorized as Minicircles (MC), which encode either a Venus fluorescent protein or a CD19-CAR together with truncated EGFR (tEGFR) as a marker, were introduced in combination with the hyperactive SB 100X transposase into primary NK cells via nucleofection. The genetically engineered NK cells were expanded using IL-15 cytokine stimulation under feeder-cell free conditions. Vector integration sites were mapped by analyzing the genomic region around each insertion site in genomic DNA from long-term cultivated gene-modified NK cells, engineered ether by lentiviral (LV) or SB-based technology. Stable gene delivery and biological activity were monitored by flow cytometry and cytotoxicity of CD19-CAR NK cells against CD19-positive ALL and CD19-negative cell line.

Robbins GM, et al. (in: Nonviral genome engineering of natural killer cells. Stem Cell Res Ther. 2021 Jun 16;12(1):350. doi: 10.1186/s13287-021-02406-6. PMID: 34134774; PMCID: PMC8207670) disclose that there are three major superfamilies of transposons commonly used for gene transfer in human cells, namely Tc1/mariner, piggyBac (PB), and hAT. The most extensively studied transposon system for gene transfer, the Sleeping Beauty (SB) transposon system, belongs to the Tc1/mariner superfamily. Since its discovery and molecular reconstruction from the genomes of salmonid fish, SB has undergone improvements through the generation of hyperactive mutants and transposon donor vector optimization. The most hyperactive variant developed so far, SB100X, is shown to have a 100-fold improvement of integration efficiency compared to the original SB transposase and is comparable to that of a viral vector system. For example, in human CD34+ hematopoietic cells, SB100X is able to achieve up to 50% integration efficiency, compared to 40% with lentiviral vectors. SB integration has a consensus target site TA. High-resolution genome-wide mapping showed SB integration favors introns, transcriptional units, upstream regulatory sequences, and microsatellite repeats.

Islam R, et al. (in: Enhancing a Natural Killer: Modification of NK Cells for Cancer Immunotherapy. Cells. 2021 Apr 29;10(5):1058. doi: 10.3390/cells10051058. PMID: 33946954; PMCID: PMC8146003) discuss strategies that are being employed to increase the number of modified NK cells and improve the ability to overcome cancer resistance and the immunosuppressive tumor microenvironment. These include the use of cytokines and synthetic compounds to bolster propagation and killing capacity, targeting immune-function checkpoints, addition of chimeric antigen receptors (CARs) to provide cancer specificity and genetic ablation of inhibitory molecules.

The present approach provides an effective and broadly applicable virus-free gene-transfer strategy for CAR NK cell generation which, due to its non-viral nature with all associated advantages and a superior safety profile, appears as a preferable alternative to viral vectors. A general advantage of MCs is the absence of antibiotic resistance genes, which eliminates the possibility of horizontal transfer to bacteria in the host and unintended integration of such genes into the host genome. With respect to the SB transposase, employing mRNA as a source for NK cells is highly attractive due to reduced cell toxicity of this approach. Furthermore, mRNA was short-lived after NK cell transposition, which reduces the risk of re-mobilization of integrated transposons and subsequent uncontrolled transpositions.

Genotoxicity by unintended activation or disruption of host genes is a serious risk for gene-modified cell products as it can lead to oncogenesis. Such effects have been reported for retrovirally transduced cell therapies and recently for non-viral transposon-based *piggyBac* (PB)-mediated generation of CAR-T cells. Importantly, the PB transposon displays an integration preference highly similar to the MLV retrovirus in terms of biased integration into TSSs. Since previous studies established a near random vector integration pattern upon SB-mediated transposition in both HSPCs and T cells, and a lower frequency of integration into highly expressed genes and cellular proto-oncogenes compared to lentiviral and retroviral vectors as well as to the PB transposon, the inventors expect a significantly lower genotoxic risk associated with the use of the SB system in therapeutic cell engineering.

To assess biosafety of the SB100X system, the inventors performed the broadest genomic analysis in genetically engineered primary NK cells so far for a CD19-CAR transposon integration and compared the insertion site distribution of SB transposons and a standard LV vector similar to those currently used in preclinical and clinical approaches. The data of the inventors demonstrate that CD19-CAR transposon integration into the NK cell genome does not have a preference for highly expressed, transcriptionally active, or potentially cancer-related genes.

CD19-CAR NK cells generated with the SB system displayed a significantly higher vector integration into genomic safe harbors than LV-transduced CAR NK cells (SB: 24% vs. LV: 14%), and a closer to random nucleotide frequency in computer-generated random positions in the genome map (Random: 43%). A similar safe integration profile in NK cells is thus expected for CAR constructs targeting other antigens upon SB-mediated transposition. Successful clinical application of SB-modified CD19-CAR cytokine-induced killer cells has been reported (Magnani CF, et al. Sleeping Beauty-engineered CAR T cells achieve antileukemic activity without severe toxicities. J Clin Invest. 2020 Nov 2;130(11):6021-6033. doi: 10.1172/JCI138473. PMID: 32780725; PMCID: PMC7598053).

The genomic integration analysis and previous work suggest SB to be a similarly efficient but safer alternative to lentiviral and retroviral vectors, with a high potential to become a favorable gene transfer system for non-viral modification. Thus, SB-generated CAR NK cells can become a favorable tool for rapid and safe immunotherapy. Especially the medical need in patients with relapsed or refractory B-ALL and the lack of allogeneic CD19-CAR T cell products makes the SB-generated CD19-CAR NK cells a promising therapeutic strategy.

Preferred is the method according to the present invention, wherein the transposable element furthermore encodes for one or more cytokines, chemokines, receptors, suicide genes and/or transfection markers.

Preferred is the method according to the present invention, wherein the CAR binds to or "targets" a tumor-associated antigen selected from BCMA, CD19 and CLEC12A, and wherein the CAR is preferably genetically modified, i.e. may comprise suitable mutations or insertions, like the modified transmembrane region as disclosed below.

Further preferred is the method according to the present invention, further comprising the step of rendering non-functional at least one immune checkpoint and/or regulatory receptor protein, such as, for example NKG2A, PD-1, and/or KIRS, wherein preferably the immune checkpoint receptor inactivation comprises a genetic deletion, a CRISPR/Cas introduced mutation, RNA interference (RNAi) and/or the recombinant expression of an immune checkpoint receptor inhibiting peptide.

These receptors are known from the art, and a number of inhibitory immunoreceptors or immune checkpoint receptors have been identified and studied in cancer in past decades, including but not limited to NKG2A, KIRS, PD-1, CTLA-4, LAG3, TIM3, TIGIT and BTLA. They are named as "immune checkpoints" referring to molecules that act as gatekeepers of immune responses (for a review, see, for example, Buckle I, Guillerey C. Inhibitory Receptors and Immune Checkpoints Regulating Natural Killer Cell Responses to Cancer. Cancers (Basel). 2021 Aug 24;13(17):4263. doi: 10.3390/cancers13174263. PMID: 34503073; PMCID: PMC8428224).

In the context of the present invention the term "inactivated" in the context of the at least one immune checkpoint receptor shall mean that the NK cell is further modified or treated in order to fully or substantially inactivate or inhibit the biological function of the at least one immune checkpoint receptor. This can be achieved using several approaches, the gene(s) of the receptor can be mutated in order to "knock-out" the function. This can be done by introducing loss of function mutation(s) (e.g., by CRISPR/Cas) and/or by introducing genetic fragments into the gene or the regulatory regions thereof. Furthermore, the function can be blocked by inactivating components of the signaling cascade of the receptor or by expressing inhibitory molecule, such as, for example, inhibitory proteins or peptides in the NK cell. The receptor may also be inactivated by inhibitory binders, like antibodies or binding fragments thereof that block the function of the receptor on the outside of the cell, and are co-provided with the NK cell treatment. Also, a combination is possible (for a review, see, for example, Buckle I, Guillerey C. Inhibitory Receptors and Immune Checkpoints Regulating Natural Killer Cell Responses to Cancer. Cancers (Basel). 2021 Aug 24;13(17):4263. doi: 10.3390/cancers13174263. PMID: 34503073; PMCID: PMC8428224).

Another aspect of the present invention relates to a recombinant CAR-NK cell, produced according to the method according to the present invention.

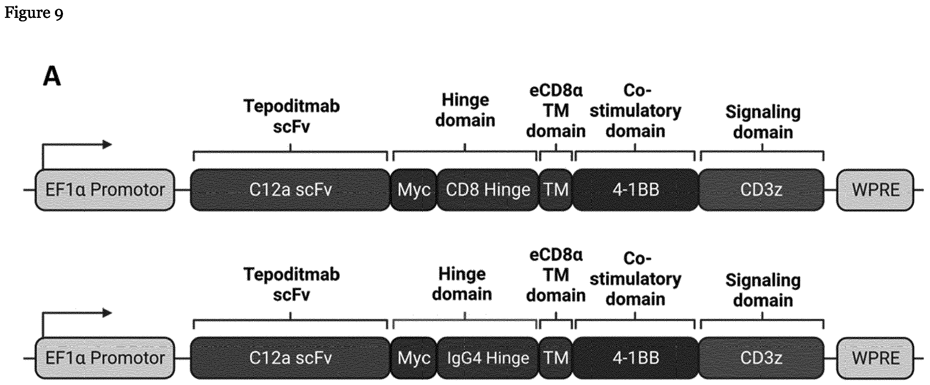

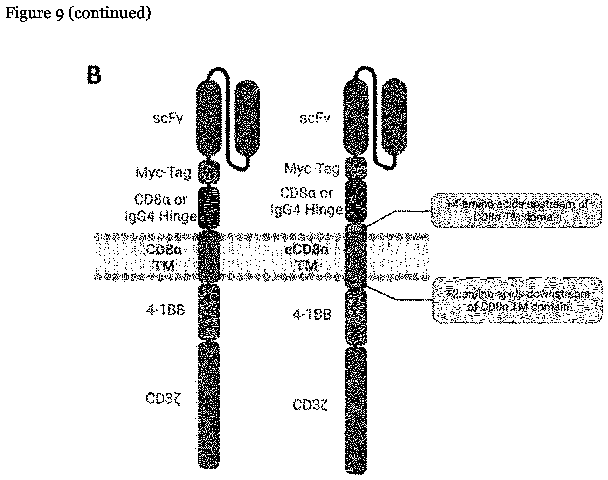

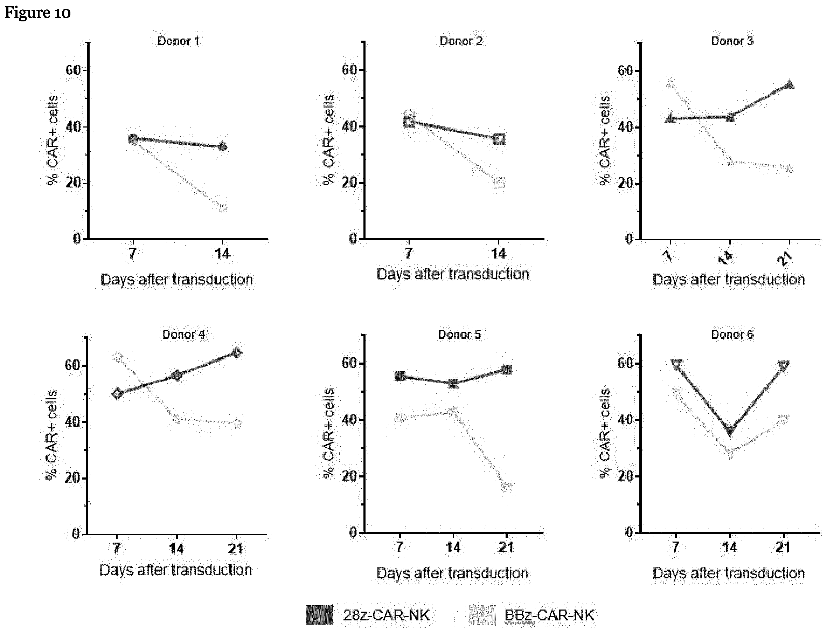

Another important aspect of the present invention relates to a CAR-construct, comprising a modified CD8α transmembrane domain comprising between 3 to 5, preferably 4 additional amino acids at the N-terminus and between 1 to 3, preferably 2 additional amino acids at the C-terminus of the CD8α transmembrane (TM) domain. It was surprisingly found, that the CD8α transmembrane domain of CAR polypeptides or fusions could be enhanced with respect to membrane stability and signal transduction, by modifying the sequence thereof as disclosed (see, for example, below and Figure 9). In view of the similarities of the CAR, this concept should be applicable both for CAR-T and for CAR-NK cell constructs (As proof of principle, the inventors efficiently transduced primary T (not shown) and NK cells (Figure 10)).

Preferred is the CAR-construct according to the present invention, comprising the amino acid sequence according to SEQ ID NO. 4 (FACDIYIWAPLAGTCGVLLLSLVITLY). In this sequence, the amino acid sequences "FACD" and "LY" were introduced at the N- and C-terminus, respectively, of the "standard" human CD8α transmembrane domain sequence. Alternatively, the transmembrane domain (TM) derived from CD28 may be similarly used. In general, any desirable target scFv can be used in the construct according to the present invention, preferably, the CAR binds to or "targets" a "foreign" or tumor-associated antigen, such as an antigen selected from BCMA, CD19 and CLEC12A.

Another aspect then relates to a nucleic acid molecule, such as a DNA or RNA, encoding for the CAR-construct according to the present invention. Another aspect then relates to an expression vector comprising said nucleic acid and expressing said nucleic acid. Respective constructs and regulatory expression sequences are well-known in the art.

Another aspect then relates to a recombinant CAR-NK cell or CAR-T-cell, comprising the CAR-construct according to the present invention or expressing the nucleic acid or expression vector according to the present invention.

Another important aspect of the present invention then relates to a recombinant CAR-NK cell, expressing a CAR binding to the antigen CLEC12A or a functional alternatively spliced transcript variant thereof, wherein at least one immune checkpoint receptor protein, such as, for example NKG2A, PD-1, and/or KIRS, is inactivated, wherein preferably the immune checkpoint receptor inactivation comprises a genetic dele tion, a CRISPR/Cas introduced mutation, RNA interference (RNAi) and/or the recombinant expression of an immune checkpoint receptor inhibiting peptide.

This aspect of the present invention relates to the example of AML-therapy using CAR-NK-cells, wherein a) CLEC12A is used as tumor-associated target, wherein the anti-CLEC12A CAR construct may be introduced into the CAR-NK-cells using either a viral, such as a lentiviral, alpharetroviral or retroviral approach or a non-viral technology such as SB (Sleeping Beauty)-transposons and wherein furthermore, as an example, b) the immune checkpoint receptor NKG2A was rendered non-functional (knocked-out or knocked down) using e.g. CRISPR/Cas9 technology. As also further explained below, in addition, c) the expression of the target CLEC12A is upregulated/stabilized using the chemotherapeutic azacitidine (AZA). This increases the cytotoxicity and reduces or even avoids the generation of resistant cancer cells ("immune escape"), and relapse. This type of CAR-NK cells provides a novel approach to the prevention or treatment of leukemia, where cells express the protein CLEC12A, such as acute myeloid leukemia (AML), and subtypes thereof. Optionally and advantageously, the prevention or treatment comprises a combination treatment with a hypomethylating agent, in particular azacitidine (AZA) and/or decitabine.

Arvindam US, et al. (in: A trispecific killer engager molecule against CLEC12A effectively induces NK-cell mediated killing of AML cells. Leukemia. 2021 Jun;35(6):1586-1596. doi: 10.1038/s41375-020-01065-5. Epub 2020 Oct 23. PMID: 33097838; PMCID: PMC8189652.9) developed a trispecific killer engager (TriKE^{™}) molecule containing a humanized anti-CD16 heavy chain camelid single domain antibody (sdAb) that activates NK cells, an IL-15 molecule that drives NK cell priming, expansion and survival, and a single-chain variable fragment (scFv) against human CLEC12A (CLEC12A TriKE). The CLEC12A TriKE induced robust NK cell specific proliferation, enhanced NK cell activation and killing of both AML cell lines and primary patient derived AML blasts in vitro while sparing healthy HSCs. Additionally, the CLEC12A TriKE was able to reduce tumor burden in pre-clinical mouse models. These findings highlight the clinical potential of the CLEC12A TriKE for the effective treatment of AML. TriKE molecules are comprised of an anti-CD16 single chain variable fragment (scFv) that activates NK cells, a mutant IL-15 molecule that adds co-stimulation and a scFv that engages cancer targets. No CAR is mentioned.

Kloess S, et al. (in: CAR-Expressing Natural Killer Cells for Cancer Retargeting. Transfus Med Hemother 2019;46:4-13. doi: 10.1159/000495771) summarize and review preclinical results of CAR-engineered NK cells using both primary human NK cells and the cell line NK-92, and provide an overview about the first clinical CAR-NK cell studies targeting haematological malignancies and solid tumours, respectively.

The present invention provides a CAR-NK cell, that is, a genetically modified NK-cell, comprising and expressing a chimeric antigen receptor (CAR). This CAR "targets" the cancer-associated antigen CLEC12A or a functional alternatively spliced transcript variant thereof on a leukemic cell through binding of the CAR binding to the target antigen expressed on the cell surface. In the context of the present invention, the term "binding" shall include all binding of the CAR to the cancer-associated antigen CLEC12A or a functional alternatively spliced transcript variant thereof that is sufficient to activate the NK cell mediate killing of leukemic cells.

In the context of the present invention the term "NK cell" shall mean CD56⁺CD3⁻ lymphocytes, including primary NK cells, and subtypes of NK cells, or NK-derived immune cell products, such as cytokine induced killer cells (CIK) or NK-92 cells as disclosed above. The majority of current clinical trials with CAR-NK cells use NK-92 cell line because of its unlimited proliferation ability in vitro and likely reduced sensitivity to repeated freeze/thaw cycles. These properties provide distinct advantages to manufacture "off-the-shelf' CAR-NK products for clinical use, with less manufacturing time and lower cost. However, NK-92 cells being a tumour cell line have inherent drawbacks including potential tumorigenicity risk, lack of CD 16 and NKp44 expression, and loss of in vivo expansion potential due to lethal irradiation needed before their infusion, making them unlikely to be an ideal cell source for CAR-NK cell therapy approaches. Human PBMCs are an important source of primary NK cells, which have been used in numerous clinical trials. Using NK cell isolation kits, a sufficient number of NK cells can be easily isolated directly from PBMCs of a healthy donor, and then stimulated and expanded in NK cell-specific expansion media with cytokines for preclinical or good manufacturing practice (GMP)-grade clinical application.

In the context of the present invention the term "cancer-associated antigen CLEC12A or a functional alternatively spliced transcript variant thereof' or "CLEC12A" relates to the protein C-type lectin domain family 12 member A, also known as CLL-1; MICL; CD371; CLL-1; DCAL-2. The gene of CLEC12A encodes a member of the C-type lectin/C-type lectin-like domain (CTL/CTLD) superfamily. Members of this family share a common protein fold and have diverse functions, such as cell adhesion, cell-cell signaling, glycoprotein turnover, and roles in inflammation and immune response. The protein encoded by this gene is a negative regulator of granulocyte and monocyte function. Several alternatively spliced transcript variants of this gene have been described, but the full-length nature of some of these variants has not been determined. The gene is closely linked to other CTL/CTLD superfamily members in the natural killer gene complex region on chromosome 12p13. The gene has currently 7 known transcripts (splice variants). The term shall also include homologs or orthologs of the protein or gene, in particular in other mammals. Preferred is the human protein having the following sequence SEQ ID NO: 1.

MSEEVTYADLQFQNSSEMEKIPEIGKFGEKAPPAPSHVWRPAALFLTLLCLLLLI
GLGVLASMFHVTLKIEMKKMNKLQNISEELQRNISLQLMSNMNISNKIRNLSTT
LQTIATKLCRELYSKEQEHKCKPCPRRWIWHKDSCYFLSDDVQTWQESKMAC
AAQNASLLKINNKNALEFIKSQSRSYDYWLGLSPEEDSTRGMRVDNIINSSAWV
IRNAPDLNNMYCGYINRLYVQYYHCTYKKRMICEKMANPVQLGSTYFREA
(SEQ ID NO. 1)

In the context of the present invention the term "functional variant thereof' in the context of the cancer-associated antigen CLEC12A shall mean a variant that is expressed by the leukemic cell in a length and/or amount to be bound by the CAR of the CAR-NK cell according to the present invention.

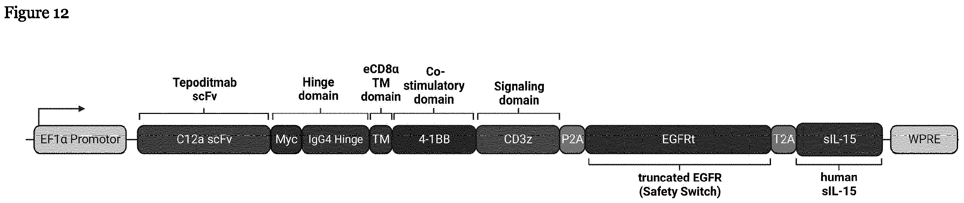

In the context of the present invention, any suitable CAR construct may be used that provides the desired NK cell activity (e.g., cancer cell killing or anti-autoimmune disease). Preferred is the CAR-NK cell according to the present invention, wherein the (CLEC12A-targeting) CAR comprises at least one scFv and/or (humanized) VHH antigen binding domain specific for the antigen (e.g. CLEC12A), a hinge domain derived from CD8α, IgG1, IgG4 or CD28, a (optimized/enhanced) transmembrane domain (TM) derived from CD28 or CD8α, one or two additional costimulatory signaling domains, such as CD28 or 4-1BB (CD137), and a CD3z signaling domain (see also Figure 9 herein). Additional modifications and/or components may be included to enhance the functionality of the modified NK cells and/or provide beneficial features for the immune cell product, such as a Myc-tag amino acid sequence, a truncated EGFR (tEGFR), receptors, cytokines or chemokines, as long as the binding and desired biological function (cancer cell killing or anti-autoimmune diseases) is not substantially impaired (see also Figure 12).

An exemplary and particularly preferred embodiment CAR molecule according to the present invention has an amino acid sequence according to SEQ ID NO: 2 and SEQ ID NO: 3, or sequences being at least 95%, preferably at least 97% and most preferably at least 99% identical thereto, wherein the enhanced CD8α transmembrane domain (eCD8α TM) is preferably according to SEQ ID NO: 4, or sequences being at least 95%, preferably at least 97% and most preferably at least 99% identical thereto. Notably, the CARs harboring the 4-1BB co-stimulatory domain (SEQ ID NO: 2 and SEQ ID NO: 3) include the optimized eCD8α transmembrane domain for enhanced membrane stability and signal transduction (see also Figure 9 herein).

Another aspect of the invention relates to a method for producing the CAR-NK cell according to the present invention, comprising the steps of introducing an antigen (here CLEC12A)-targeting CAR or a functional alternatively spliced transcript variant thereof using a viral or non-viral gene transfer system, and genetically rendering non-functional one or more immune checkpoint receptors. As mentioned above, preferred is the method according to the present invention, wherein the gene transfer system is either a lentiviral, alpharetroviral or retroviral system or a non-viral technology such as a transposon-based system utilizing a plasmid or Minicircle (MC) vector carrying the gene of interest (GOI), i.e. the CAR.

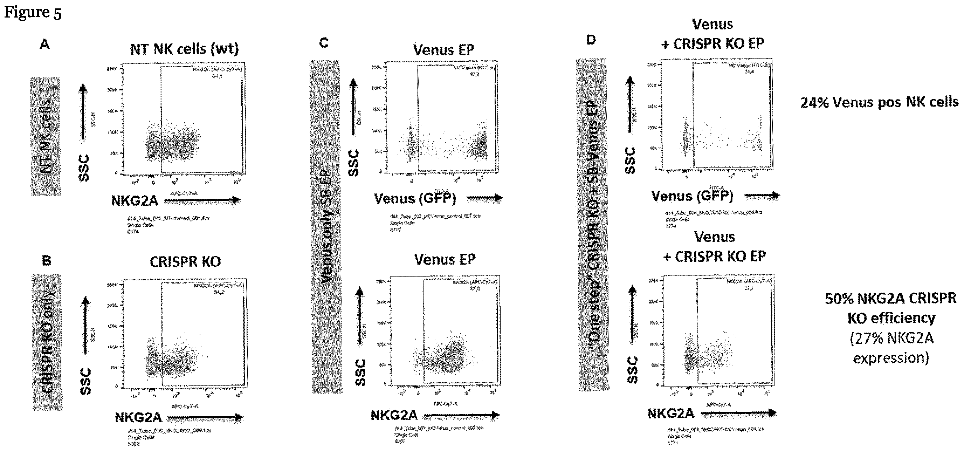

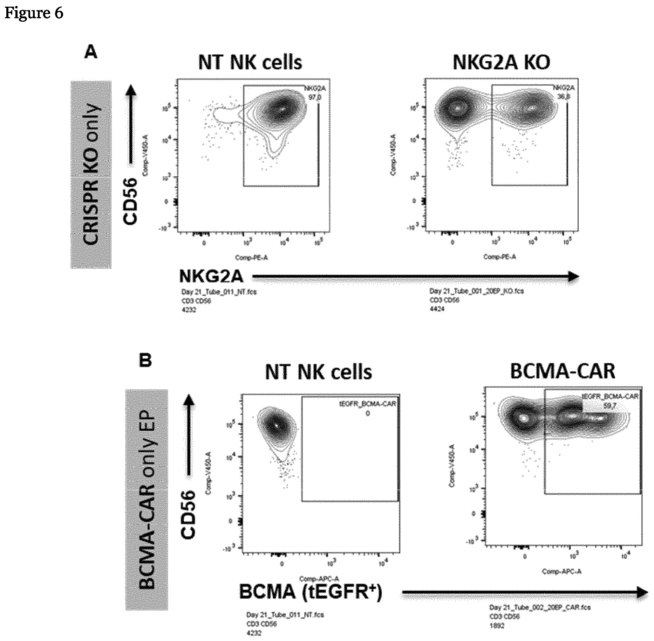

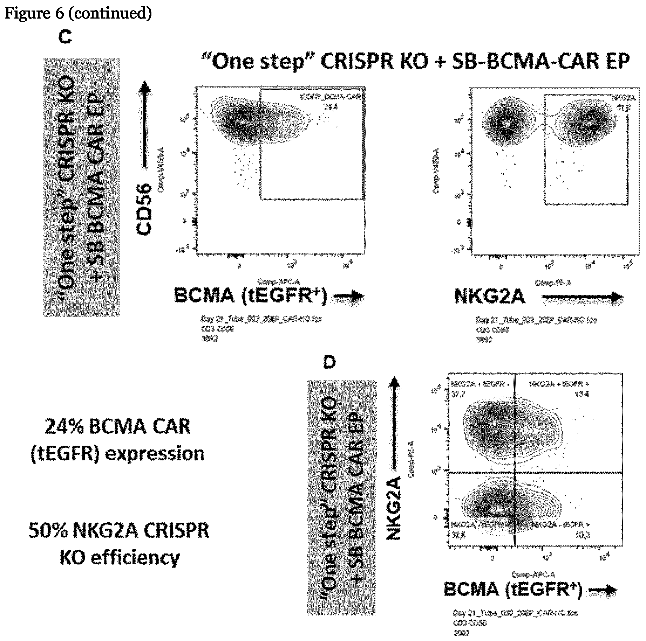

In the method according to the present invention, the introduction of the CAR genetic construct into the NK cell, and the immune checkpoint receptor inactivation in the NK cell are accomplished in a single ("one-step") or successive genetic engineering steps, i.e. are performed together. Examples for such one-step nucleofection protocols are the methods are described herein. Figure 5 shows representative FACS-plots of NK cells after double modification by "one-step" nucleofection using SB/MC (Venus protein) and RNP-based CRISPR/Cas9 knockout (KO) nucleofection (EP). Figure 6 shows representative FACS-plots of NK cells after double modification by "one-step" nucleofection using SB/MC (BCMA-CAR) and RNP-based CRISPR/Cas9 knockout (KO) nucleofection (EP). As described above, in method according to the present invention, the immune checkpoint receptor inactivation may comprise genetic deletion, CRISPR/Cas introduced mutation, RNA interference (RNAi) and/or recombinant expression of an inhibiting peptide, as well as the provision of immune checkpoint receptor inactivating compounds.

In the context of the present invention, preferred embodiments of amino acid sequences for the CLEC12A-CAR construct, more preferably including the eCD8α transmembrane domain as designed are as follows:
CLEC12A.CD8αh.eCD8αTM.BBz; amino acid sequence 
DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPPTFGQGTKVEIKGGG
GSGGGGSGGGGSQVQLVQSGAEVKKPGASVKVSCKASGYTFTSYYMHWVRQ
APGQGLEWMGIINPSGGSTSYAQKFQGRVTMTRDTSTSTVYMELSSLRSEDTA
VYYCAKGTTGDWFDYWGQGTLVTVSSEQKLISEEDLALSNSIMYFSHFVPVFL
PAKPTTTPAPRPPTPAPTIASQPLSLRPEASRPAAGGAVHTRGLDFACDIYIW
APLAGTCGVLLLSLVITLYKRGRKKLLYIFKQPFMRPVQTTQEEDGCSCRFPEEE
EGGCELRVKFSRSADAPAYQQGQNQLYNELNLGRREEYDVLDKRRGRDPEMGGKP
RRKNPQEGLYNELQKDKMAEAYSEIGMKGERRRGKGHDGLYQGLSTATKDTYDAL
HMQALPPR (Anti-CLEC12A scFv - Myc tag (underlined) - CD8α hinge domain (bold)
 - enhanced CD8α transmembrane domain (eCD8α TM) (double underlined) - 4-1BB (CD137) costimulatory domain - CD3 zeta domain (*italic*)*,* SEQ ID NO: 2).
CLEC12A.IgG4h.eCD8αTM.BBz; amino acid sequence 
DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPPTFGQGTKVEIKGGG
GSGGGGSGGGGSQVQLVQSGAEVKKPGASVKVSCKASGYTFTSYYMHWVRQ
APGQGLEWMGIINPSGGSTSYAQKFQGRVTMTRDTSTSTVYMELSSLRSEDTA
VYYCAKGTTGDWFDYWGQGTLVTVSSEQKLISEEDLESKYGPPCPSCPFACDI
YIWAPLAGTCGVLLLSLVITLYKRGRKKLLYIFKQPFMRPVQTTQEEDGCSCRF
PEEEEGGCELRVKFSRSADAPAYQQGQNQLYNELNLGRREEYDVLDKRRGRDPEM
 
GGKPRRKNPQEGLYNELQKDKMAEAYSEIGMKGERRRGKGHDGLYQGLSTATKDT
YDALHMQALPPR (Anti-CLEC12A scFv - Myc tag (underlined) – IgG4 hinge domain
 **(bold)** - enhanced CD8α transmembrane domain (eCD8α TM) (double underlined) - 4-1BB (CD137) costimulatory domain - CD3 zeta domain (*italic*)*,* SEQ ID NO: 3)

In the CAR-NK cell according to the present invention, one or more immune checkpoint receptor, such as, for example NKG2A, PD-1, and/or KIRS, is/are inactivated. These receptors are known from the art, and a number of inhibitory immunoreceptors or immune checkpoint receptors have been identified and studied in cancer in past decades, including but not limited to NKG2A, KIRS, PD-1, CTLA-4, LAG3, TIM3, TIGIT and BTLA. They are named as "immune checkpoints" referring to molecules that act as gatekeepers of immune responses (for a review, see, for example, Buckle I, Guillerey C. Inhibitory Receptors and Immune Checkpoints Regulating Natural Killer Cell Responses to Cancer. Cancers (Basel). 2021 Aug 24;13(17):4263. doi: 1 0.3390/cancers 13 174263. PMID: 34503073; PMCID: PMC8428224).

In the context of the present invention the term "inactivated" in the context of the at least one immune checkpoint receptor shall mean that the NK cell is further modified or treated in order to fully or substantially inactivate or inhibit the biological function of the at least one immune checkpoint receptor. This can be achieved using several approaches, the gene(s) of the receptor can be mutated in order to "knock-out" the function. This can be done by introducing loss of function mutation(s) (e.g., by CRISPR/Cas) and/or by introducing genetic fragments into the gene or the regulatory regions thereof. Furthermore, the function can be blocked by inactivating components of the signaling cascade of the receptor or by expressing inhibitory molecule, such as, for example, inhibitory proteins or peptides in the NK cell. The receptor may also be inactivated by inhibitory binders, like antibodies or binding fragments thereof that block the function of the receptor on the outside of the cell and are co-provided with the NK cell treatment. Also, a combination is possible (for a review, see, for example, Buckle I, Guillerey C. Inhibitory Receptors and Immune Checkpoints Regulating Natural Killer Cell Responses to Cancer. Cancers (Basel). 2021 Aug 24;13(17):4263. doi: 10.3390/cancers13174263. PMID: 34503073; PMCID: PMC8428224).

Another aspect of the present invention relates to a pharmaceutical composition, comprising the CAR-NK cell according to the present invention, the nucleic acid or the expression vector according to the present invention, or the CAR-NK cell or CAR-T-cell according to the present invention, together with at least one pharmaceutically acceptable diluent or carrier.

Holzinger A, and Abken H (in: Treatment with Living Drugs: Pharmaceutical Aspects of CAR T Cells. Pharmacology. 2022;107(9-10):446-463. doi: 10.1159/000525052. Epub 2022 Jun 13. PMID: 35696994) discuss pharmaceutical aspects, the regulatory requirements for CAR T cell manufacturing, and how CAR T cell pharmacokinetics are connected with the clinical outcome. They also discuss NK cell therapeutics.

Lu H, et al. (From CAR-T Cells to CAR-NK Cells: A Developing Immunotherapy Method for Hematological Malignancies. Front Oncol. 2021 Aug 6;11:720501. doi: 10.3389/fonc.2021.720501. PMID: 34422667; PMCID: PMC8377427) discuss the current status and applications of CAR-NK cells in hematological malignancies, as well as the unique advantages of CAR-NK cells compared with CAR-T cells. They also discuss challenges and prospects regarding clinical applications of CAR-NK cells.

The term "pharmaceutically or therapeutically acceptable excipient or carrier" refers to a solid or liquid filler, diluent or encapsulating substance which does not interfere with the effectiveness or the biological activity of the active ingredients and which is not toxic to the host, which may be either humans or animals, to which it is administered. Depending upon the particular route of administration, a variety of pharmaceutically acceptable carriers such as those well known in the art may be used. Non-limiting examples include sugars, starches, cellulose and its derivatives, malt, gelatin, talc, calcium sulfate, vegetable oils, synthetic oils, polyols, alginic acid, phosphate buffered solutions, emulsifiers, isotonic saline, and pyrogen-free water. Pharmaceutically acceptable carriers or excipients also include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal Si0₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for the compounds according to the present invention, and will readily be able to choose suitable pharmaceutically acceptable carriers or excipients, depending, e.g., on the formulation and administration route of the pharmaceutical composition.

All suitable modes of administration are contemplated according to the invention, preferred is infusion. Administration of the medicament may be via oral, subcutaneous, direct intravenous, slow intravenous infusion, continuous intravenous infusion, intravenous or epidural patient controlled analgesia (PCA and PCEA), intramuscular, intrathecal, epidural, intracistemal, intraperitoneal, transdermal, topical, buccal, sublingual, transmucosal, inhalation, intra-atricular, intranasal, rectal or ocular routes, abuse deterrent and abuse resistant formulations, sterile solutions suspensions and depots for parenteral use, and the like, administered as immediate release, sustained release, delayed release, controlled release, extended release and the like.

In addition to the aforementioned compounds of the invention, the pharmaceutical composition can contain two or more compounds according to the present invention and also other therapeutically active substances.

The dosage of the pharmaceutical composition according to the present invention can be appropriately selected according to the route of administration, the subject to be administered, the target disease and its severity, age, sex weight, individual differences and disease state. Dosage may be repeated several times a day.

Another aspect of the present invention relates to a method for producing the pharmaceutical composition according to the present invention comprising the steps of providing at least one of the CAR-NK cell according to the present invention the nucleic acid or the expression vector according to the present invention, or the CAR-NK cell or CAR-T-cell according to the present invention, admixing it with at least one pharmaceutically acceptable carrier and/or excipient. The medicament may be formulated in discrete dosage units and can be prepared by any of the methods well known in the art of pharmacy. The pharmaceutical composition of the present invention can be formulated using methods known in the art to provide rapid, sustained or delayed release of the active ingredient after administration to a mammal.

El Khawanky N, et al. (in: Demethylating therapy increases anti-CD123 CAR T cell cytotoxicity against acute myeloid leukemia. Nat Commun. 2021 Nov 8;12(1):6436. doi: 10.1038/s41467-021-26683-0. PMID: 34750374; PMCID: PMC8575966) developed third-generation anti-CD123 CAR T cells with a humanized CSL362-based scFv and a CD28-OX40-CD3ζ intracellular signaling domain. They found that CD123 expression on leukemia cells increases upon 5-azacytidine (azacitidine, AZA) treatment. AZA treatment of leukemia-bearing mice causes an increase in CTLA-4 negative anti-CD123 CAR T cell numbers following infusion. Functionally, the CTLA-4 negative anti-CD123 CAR T cells exhibit superior cytotoxicity against AML cells, accompanied by higher TNFα production and enhanced downstream phosphorylation of key T cell activation molecules. AZA increases the immunogenicity of AML cells, enhancing recognition and elimination of malignant cells by highly efficient CTLA-4 negative anti-CD123 CAR T cells.

Indeed, engineered T cells equipped with a synthetic chimeric antigen receptor (CAR) have demonstrated remarkable efficacy in the treatment of hematological malignancies. Currently, four CD19-targeting CAR T cell products are approved for treatment of acute B cell leukemia and lymphoma (B-ALL/ -CLL / -NHL) by the FDA and EMA. Although immunotherapies such as blinatumomab or CD19-directed CAR-T cells can lead to an improved B-ALL outcome (Kantarjian H, et al. Blinatumomab versus Chemotherapy for Advanced Acute Lymphoblastic Leukemia. N Engl J Med. 2017 Mar 2;376(9):836-847. doi: 10.1056/NEJMoa1609783. PMID: 28249141; PMCID: PMC5881572.), many patients relapse or are refractory to those therapies and have a poor prognosis.

Moreover, T cells can also cause various side effects, which include cytokine release syndrome (CRS), immune effector cell-associated neurotoxicity syndrome (ICANS) and, in the case of donor-derived allogeneic T cells, graft-versus-host disease (GvHD). Also, the potential for insertional oncogenesis of viral vectors remains a safety concern, and costs and regulatory requirements of autologous CAR-T cell products limit broad clinical translation (Alnefaie A, et al. Chimeric Antigen Receptor T-Cells: An Overview of Concepts, Applications, Limitations, and Proposed Solutions. Front Bioeng Biotechnol. 2022 Jun 22;10:797440. doi: 10.3389/fbioe.2022.797440. PMID: 35814023; PMCID: PMC9256991). CAR-engineered natural killer (NK) cells hold the potential to overcome these limitations, in particular if genetically modified by use of a non-viral gene transfer system. NK cells are known for their high intrinsic cytotoxic capacity, safety, and efficacy. In a recently reported clinical trial, NK cells modified with a CD19-CAR showed a high response rate of over 70% in patients with B-CLL leukemia and lymphoma, without signs of CRS or ICANS which are frequently observed upon CD19-CAR T cell therapy (Liu E, et al. Use of CAR-Transduced Natural Killer Cells in CD19-Positive Lymphoid Tumors. N Engl J Med. 2020 Feb 6;382(6):545-553. doi: 10.1056/NEJMoa1910607. PMID: 32023374; PMCID: PMC7101242). Furthermore, due to their intrinsic natural cytotoxicity, gene-modified NK cells can recognize cancer cells through alternative mechanisms in addition to CAR-specific binding to tumor antigens. Thus, NK cells have the potential to also kill leukemia cells that have lost CD19 antigen expression, which is still a problem with CD19-CAR T cell therapy (Reindl LM, et al. Immunotherapy with NK cells: recent developments in gene modification open up new avenues. Oncoimmunology. 2020 Sep 2;9(1):1777651. doi: 10.1080/2162402X.2020.1777651. PMID: 33457093; PMCID: PMC7781759).

In contrast to the above T-cell related publications, CAR NK cell therapies are thus emerging as a promising approach for cell-based immunotherapies that can be applied for the treatment of advanced malignancies without serious adverse effects. Nevertheless, NK cells are typically more difficult to transfect than T cells, most likely due to enhanced resistance to the uptake of foreign nucleic acids as a first-line defense against viral pathogens. Although the efficiency of lenti- and retroviral engineering of CAR NK cells has improved in recent years and led to promising results (Reindl LM, et al. Immunotherapy with NK cells: recent developments in gene modification open up new avenues. Oncoimmunology. 2020 Sep 2;9(1):1777651. doi: 10.1080/2162402X.2020.1777651. PMID: 33457093; PMCID: PMC7781759; Wendel P, et al. Arming Immune Cells for Battle: A Brief Journey through the Advancements of T and NK Cell Immunotherapy. Cancers (Basel). 2021 Mar 23;13(6):1481. doi: 10.3390/cancers13061481. PMID: 33807011; PMCID: PMC8004685), non-viral NK cell can also be applied for NK cells (Schmidt P, Raftery MJ, Pecher G. Engineering NK Cells for CAR Therapy-Recent Advances in Gene Transfer Methodology. Front Immunol. 2021 Jan 7;11:611163. doi: 10.3389/fimmu.2020.611163. PMID: 33488617; PMCID: PMC7817882).

Yet another aspect of the present invention relates to the CAR-NK cell targeting the cancer-associated antigen CLEC12A or a functional alternatively spliced transcript variant thereof according to the present invention the CAR-NK cell according to the present invention, the nucleic acid or the expression vector according to the present invention, the CAR-NK cell or CAR-T-cell according to the present invention or the pharmaceutical composition according to the present invention for use in medicine, in particular for use in the prevention or treatment of cancer, such as leukemia, in a patient. Preferably, the prevention or treatment comprises a combination treatment with a hypomethylating agent.

By "treatment" or "treating" is meant any treatment of a disease or disorder, in a patient, including: preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms. By "amelioration" is meant the prevention, reduction or palliation of a state, or improvement of the state of a subject; the amelioration of a stress is the counteracting of the negative aspects of a stress. Amelioration includes, but does not require complete recovery or complete prevention of a stress.

Preferred is the CAR-NK cell according to the present invention, the nucleic acid or the expression vector according to the present invention, the CAR-NK cell or CAR-T-cell according to the present invention or the pharmaceutical composition according to the present invention for use according to the present invention, wherein the leukemia is selected from the group consisting of leukemic cells expressing CLEC12A, acute myeloid leukemia (AML), and subtypes thereof (for the subtypes, see, for example, https://www.cancer.org/cancer/acute-myeloid-leukemia/detection-diagnosis-staging/how-classified.html).

It is to be understood that the present compound and/or a pharmaceutical composition comprising the present compound is for use to be administered, or administered as a combination treatment, to a patient. The term "administering" means administration of a sole therapeutic agent (the inventive NK cells) or in combination with another therapeutic agent (e.g. AZA). It is thus envisaged that the pharmaceutical composition of the present invention is employed in co-therapy approaches, i.e. in co-administration with other medicaments or drugs and/or any other therapeutic agent, preferably a hypomethylating agent, such as AZA, which are beneficial in the context of the methods of the present invention. Nevertheless, the other medicaments or drugs and/or any other therapeutic agent can be administered separately from the compound for use, if required, as long as they act in combination (i.e. directly and/or indirectly, preferably synergistically) with the present compound(s) (for use). Other compounds to be co-administered comprise commonly known anti-leukemia and anti-AML treatments.

According to the present invention, a "patient" can be preferably selected from a mouse, rat, cat, dog, rabbit, goat, sheep, horse, camel, lama, cow, monkey, a farm animal, a sport animal, and a pet, and a human.

In another aspect of the CAR-NK cell according to the present invention, the nucleic acid or the expression vector according to the present invention, the CAR-NK cell or CAR-T-cell according to the present invention or the pharmaceutical composition according to the present invention for use according to the present invention the prevention and/or treatment further comprises detecting and/or monitoring of the success of a treatment in said patient. This monitoring usually is performed on a biologically sample taken from the patient, and comprises commonly known tests with respect to the progress/remission of the leukemia, such as AML, for example antibody based, PCR based, and the like. The tests are repeated over time, and can be compared to control samples and/or samples taken earlier from the patient. The results help the attending physician to maintain or modify the course of a treatment, usually based on the severity of the clinical symptoms of the cancer/leukemia or autoimmune diseases as treated.

Further preferred is the CAR-NK cell according to the present invention, the nucleic acid or the expression vector according to the present invention, the CAR-NK cell or CAR-T-cell according to the present invention or the pharmaceutical composition according to the present invention for use according to the present invention, wherein the prevention or treatment comprises a combination treatment with a hypomethylating agent, such as, for example, selected from azacitidine (AZA) and/or decitabine (see, for example, Contieri B, Duarte BKL, Lazarini M. Updates on DNA methylation modifiers in acute myeloid leukemia. Ann Hematol. 2020 Apr;99(4):693-701. doi: 10.1007/s00277-020-03938-2. Epub 2020 Feb 6. PMID: 32025842).

Yet another aspect of the present invention the relates to the CAR-NK cell for use according to the present invention, wherein said CAR-NK cell is used as the pharmaceutical composition according to the present invention (see above).

In another aspect thereof, the present invention provides a method for preventing or treating cancer, such as leukemia, or autoimmune diseases in a patient, comprising administering an effective amount of the CAR-NK cell according to the present invention, the nucleic acid or the expression vector according to the present invention, the CAR-NK cell or CAR-T-cell according to the present invention or the pharmaceutical composition according the present invention to said patient. Preferably, the prevention or treatment comprises a combination treatment with an effective amount of a hypomethylating agent, such as, for example, selected from azacitidine (AZA) or decitabine. In general, the same details and considerations apply for the method as for the medical use as above.

Preferred is the method according to the present invention, wherein the leukemia is selected from the group consisting of leukemic cells expressing CLEC12A, acute myeloid leukemia (AML), and subtypes thereof. Further preferred is the method according to the present invention, wherein said CAR-NK cell is administered as the pharmaceutical composition according to the present invention.

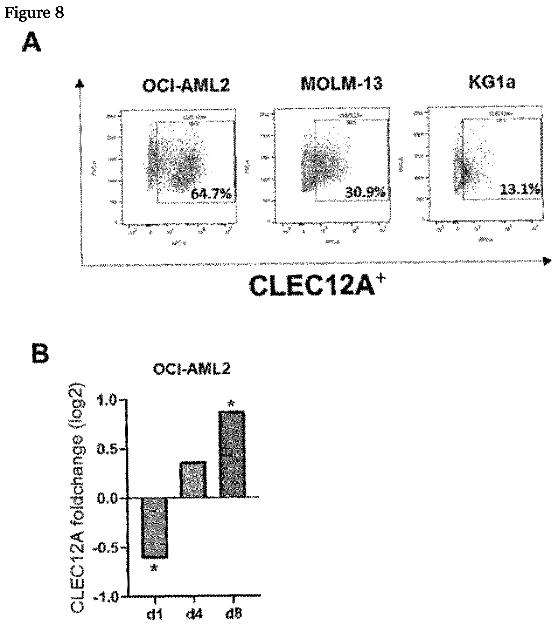

In the context of the present invention, a preferred and specific CLEC12A-targeting CAR-NK cell was designed and CLEC12A expression was upregulated/extended in parallel by the use of a hypomethylating agent, such as AZA. This approach was based on the findings that treatment with AZA upregulates CLEC12A expression (Figure 8) and enhances sensitivity of AML cells to CAR-T cell mediated cytotoxicity irrespective of the target (El Khawanky N, et al. Demethylating therapy increases anti-CD123 CAR T cell cytotoxicity against acute myeloid leukemia. Nat Commun. 2021; 12:6436).

The inventors in a preclinical xenografted mouse model observed that the leukemia burden was lowest in the CD123-CAR-T cell / AZA combination group as compared to all other groups. Consistent with the AML *in vivo* elimination, the inventors also observed that the AML bearing mice that received CD123-targeting CAR-T cells with AZA exhibited superior survival compared to AZA alone or CAR-T cells alone or AZA with non-transduced T cells (NTD Tc). The number of residual human CD45⁺ and MOLM-13 leukemia cells found in the bone marrow (BM) of mice that received CD123-CAR-T along with AZA was significantly lower compared to groups receiving AZA alone or CAR-T cells alone or AZA with NTD Tc.

Based on these findings, in order to overcome the disadvantages of using CD123 as a target as explained herein, the inventors defined CLEC12A as highly promising target antigen due to its expression on more than 90% of leukemic blasts from AML patients as well as on OCI-AML2 and MOLM-13 cells (Figure 8). Surprisingly, exposure of OCI-AML cells to AZA for 4 or 8 days led to significant CLEC12A upregulation (Figure 8).

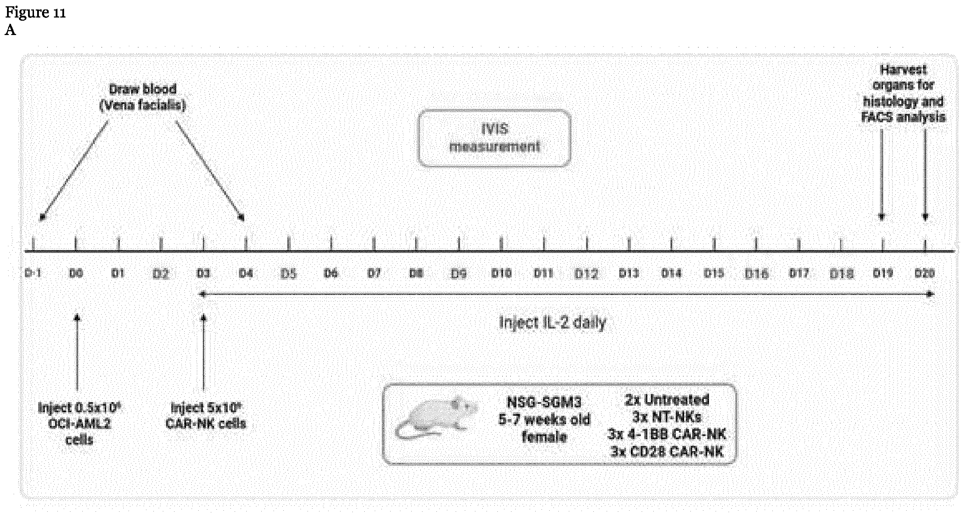

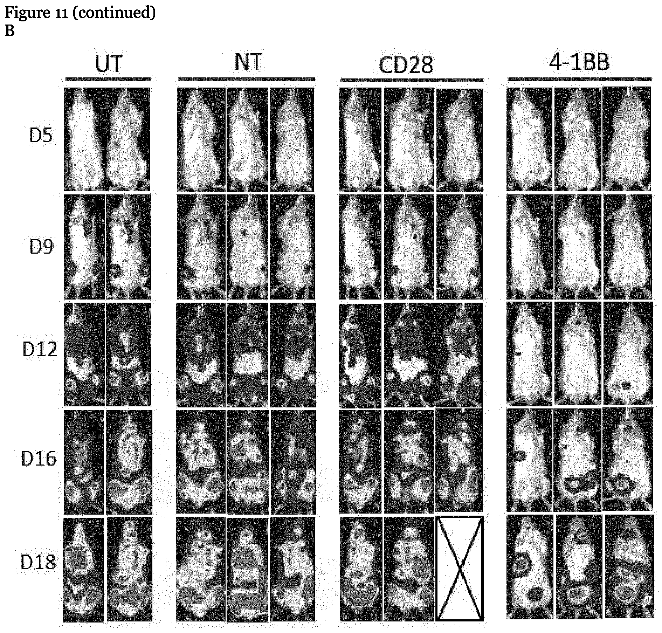

CLEC12A is not expressed on HSCs or other tissues. The inventors designed a specific CLEC12A-targeting CAR with a CD8 or IgG4 hinge domain, an (improved) eCD8α or CD28 TM domain, a CD28 or 4-1BB co-stimulatory domain, and a CD3z signaling domain (Figure 9). As proof-of-principle, the inventors efficiently transduced primary T and NK cells (Figure 10) and further addressed CLEC12A-targeting CAR-T/-NK cell mediated lysis of different CLEC12A-expressing AML cell lines *in vitro.* The inventors observed enhanced killing capacity against the CLEC12A^{high}-expressing OCI-AML2 and CLEC12A^{dim}-expressing MOLM-13 cell line, but not against CLEC12A^{low}-expressing KG1a cells in long- (24 hours) and short-term (4 hours) cytotoxicity assay. CAR expression has also been demonstrated for both the CD28-based CLEC12A-CAR or the 4-1BB-based CLEC12A-CAR-NK cells. Furthermore, cytotoxicity has been demonstrated *in vitro* and *in vivo* in an AML xenograft model (Figure 11).

The state-of-the-art method to genetically engineer primary CAR expressing cells is the use of lentiviral or gamma-retroviral vectors, but GMP-production of such viral vectors is a time consuming and costly procedure. Non-viral gene transfer systems on the other hand can open new avenues for cell-based cancer immunotherapy, such as the *Sleeping Beauty* (SB) transposon gene transfer system, which mainly consists of a DNA vector encoding the transgene flanked by inverted terminal repeats (ITRs) which contain binding sites for the transposase. The SB platform was the first ever shown to be capable of efficient transposition in vertebrate cells, opening new avenues for genetic engineering in gene therapy. Recent developments that address both the efficiency and safety of SB gene delivery are the combination of an mRNA-encoded SB transposase with Minicircle (MC) vectors that encode the transgene. Currently a total of 14 active clinical trials in gene therapy make use of SB gene transfer technology with additional ones in the planning (recently reviewed in Amberger M, Ivics Z. Latest Advances for the Sleeping Beauty Transposon System: 23 Years of Insomnia but Prettier than Ever: Refinement and Recent Innovations of the Sleeping Beauty Transposon System Enabling Novel, Nonviral Genetic Engineering Applications. Bioessays. 2020 Nov;42(11):e2000136. doi: 10.1002/bies.202000136. Epub 2020 Sep 16. PMID: 32939778).

SB has made a successful clinical debut in two studies of CD19-targeting CAR-T cell therapy. These clinical studies showed that the administration of SB-engineered CD19-specific CAR-T cells is safe and augments the graft-versus-leukemia effect in patients after allo-HCT. There is a wide range of human cell types that are amenable to SB transposon-based genome engineering. The use of SB-based gene transfer has been shown to provide a stable and long-lasting CAR expression in NK cells. Furthermore, the resulting CD19-targeting CAR-NK cells demonstrated a high cytotoxic capacity *in vitro* and *in vivo.*

Locus-specific engineering of human cells can be achieved with genome editing tools, such as TALENs and the CRISPR/Cas system. In the context of immune cells, designer nucleases have been successfully applied in CAR-T cells to disrupt immune checkpoint encoding genes, such as PD-1 and LAG3, in order to increase their anti turn oral activity (Mussolino C, et al. Genome and Epigenome Editing to Treat Disorders of the Hematopoietic System. Hum Gene Ther. 2017;28(11):1105-1115). Furthermore, genome editing has been used to manufacture so-called universal or off-the-shelf CAR-T cell products by knocking out *inter alia* the loci encoding the T cell receptor (Mussolino C, et al. Genome and Epigenome Editing to Treat Disorders of the Hematopoietic System. Hum Gene Ther. 2017;28(11):1105-1115). Various activity enhanced and/or universal CAR-T cells have been successfully applied in clinic settings (Stadtmauer EA, et al. CH. CRISPR-engineered T cells in patients with refractory cancer. Science. 2020;367(6481)).

Because gene editing can be associated with off-target effects that must be carefully addressed before clinical application of the modified cells, the inventors established CAST-Seq, a genome-wide method to identify and quantify CRISPR/Cas and TALEN-induced chromosomal aberrations (Turchiano G, et al. Quantitative evaluation of chromosomal rearrangements in gene-edited human stem cells by CAST-Seq. Cell Stem Cell. 2021;28(6):1136-1147 e1135.). CAST-Seq is performed directly on genomic DNA of the clinically applied, gene-edited cells. The method is consequently particularly relevant for genome editing in a therapeutic context in order (i) to identify the best-suited CRISPR/Cas nuclease to modify a particular target locus, and (ii) to enable a thorough preclinical risk assessment of the gene-edited NK cell product before clinical translation.

Recently, the inventors have succeeded in generating CD33-targeting CAR-NK cells that show high cytotoxicity against AML cell lines and primary AML blasts (Albinger N, et al. Primary CD33-targeting CAR-NK cells for the treatment of acute myeloid leukemia. Blood Cancer J. 2022;12(4):61). A single treatment with CD33-CAR-NK cells led to reduced AML-progression in Luc⁺-OCI-AML2 xenografted NSG mice. The above-mentioned advances in genetic engineering can be used to enhance further the function of CD33-targeting CAR-NK cells by knockout of immune suppressive factors, such as NKG2A or PD1 (Figure 5A). The inventors' data demonstrate that highly therapy-resistant tumor entities, such as the tumors based on the cell line AML-2, can be efficiently eliminated by CD33-CAR expressing and NKG2A-deficient NK cells (Figure 5B-C).

Overall, the inventors' data demonstrate the successful establishment of (i) combination therapy that combines hypomethylating AZA with CAR immune cells, (ii) highly efficient and stable incorporation of CAR transgenes in NK cells, and (iii) CRISPR-Cas9 mediated knockout of key inhibitory molecules in NK cells for improved activity.

AML is the most common form of leukemia in adults with a high morbidity and mortality rate. Especially patients who are not eligible for allo-HCT are prone to therapy-resistance and early relapse. Therefore, the development of new treatment concepts, in particular immunotherapy approaches with minimal side effects, is of urgent medical need. Recent findings demonstrated that combinatorial approaches of hypomethylating agents with CD123-targeting CAR-T cells are promising. However, on-target/off-tumor effects of CD123-targeting immune cells and immune suppression by components of the tumor microenvironment might complicate clinical translation. Furthermore, the use of CAR-T cells in clinical application is still mostly restricted to the generation of autologous cell products, which might fail in case of intensively pre-treated patients. In a preferred embodiment, the present invention aims to overcome these hurdles by the use of (a) non-viral SB transposon and CRISPR-Cas9-mediated genome engineering in allogeneic NK cells to manufacture a CLEC12A-targeting CAR-immune therapeutic product with enhanced anti-tumor activity, and (b) combine CAR/KO-NK cell therapy with an AZA-based induction protocol in AML.

The present invention relates to the following items:
Item 1. A method for producing a CAR-NK cell expressing an antigen-targeting chimeric antigen receptor (CAR) or a functional alternatively spliced transcript variant thereof, comprising the steps of a) providing primary NK cells, b) pretreating the primary NK cells for about 2 days in a suitable medium comprising about 10 ng/ml IL15 or an equivalent amount of IL2, c) nucleofecting the cells with i) at least one Minicircle DNA encoding a transposon cassette element comprising a nucleic acid encoding the CAR or the functional alternatively spliced transcript variant thereof, and ii) a nucleic acid encoding at least one Sleeping Beauty (SB 100X) transposase using a suitable nucleofection program, such as, for example, DO100 or DK100, and d) expanding the nucleofected cells in a suitable medium comprising about 10 ng/ml IL15 or an equivalent amount of IL2.
Item 2. The method according to Item 1, wherein the transformation efficiency of the NK cells is at least 30%, preferably at least 40%, and more preferably at least 50%, and wherein preferably a stable integration is achieved.
Item 3. The method according to Item 1 or 2, wherein the nucleic acid is an mRNA molecule, such as an SNIM mRNA.
Item 4. The method according to any one of Items 1 to 3, wherein the transposable element furthermore encodes for one or more cytokines, chemokines, receptors, suicide genes and/or transfection markers.
Item 5. The method according to any one of Items 1 to 4, wherein the CAR binds to an antigen selected from BCMA, CD19 and CLEC12A, and wherein the CAR is preferably genetically modified.
Item 6. The method according to any one of Items 1 to 5, further comprising the step of rendering non-functional at least one immune checkpoint and/or regulatory receptor protein, such as, for example NKG2A, PD-1, and/or KIRS, wherein preferably the immune checkpoint receptor inactivation comprises a genetic deletion, a CRISPR/Cas introduced mutation, RNA interference (RNAi) and/or the recombinant expression of an immune checkpoint receptor inhibiting peptide.
Item 7. A recombinant CAR-NK cell, produced according to the method according to any one of Items 1 to 6.
Item 8. A CAR-construct, comprising a modified CD8α transmembrane domain comprising between 3 to 5, preferably 4 additional amino acids at the N-terminus and between 1 to 3, preferably 2 additional amino acids at the C-terminus of the CD8α transmembrane (TM) domain.
Item 9. The CAR-construct according to Item 8, comprising the amino acid sequence according to SEQ ID No. 4.
Item 10. The CAR-construct according to Item 8 or 9, wherein the CAR binds to an tumor-associated antigen, such as an antigen selected from BCMA, CD19 and CLEC12A.
Item 11. A nucleic acid encoding for the CAR-construct according to any one of Items 8 to 10 or an expression vector comprising said nucleic acid.
Item 12. A recombinant CAR-NK cell or CAR-T-cell, comprising the CAR-construct according to any one of Items 8 to 10 or expressing the nucleic acid or expression vector according to Item 11.
Item 13. A recombinant CAR-NK cell, expressing a CAR binding to the antigen CLEC12A or a functional alternatively spliced transcript variant thereof, wherein at least one immune checkpoint receptor protein, such as, for example NKG2A, PD-1, and/or KIRS, is inactivated, wherein preferably the immune checkpoint receptor inactivation comprises a genetic deletion, a CRISPR/Cas introduced mutation, RNA interference (RNAi) and/or the recombinant expression of an immune checkpoint receptor inhibiting peptide.
Item 14. A pharmaceutical composition, comprising the CAR-NK cell according to Item 7 or 13, the nucleic acid or the expression vector according to Item 11, or the CAR-NK cell or CAR-T-cell according to Item 12, together with at least one pharmaceutically acceptable diluent or carrier.
Item 15. The CAR-NK cell according to Item 7 or 13, the nucleic acid or the expression vector according to Item 11, the CAR-NK cell or CAR-T-cell according to Item 12 or the pharmaceutical composition according to Item 14 for use in medicine, in particular for use in the prevention or treatment of cancer, such as leukemia, or autoimmune diseases in a patient.
Item 16. The CAR-NK cell according to Item 7 or 13, the nucleic acid or the expression vector according to Item 11, the CAR-NK cell or CAR-T-cell according to Item 12 or the pharmaceutical composition according to Item 14 for use according to Item 15, wherein the leukemia is selected from the group consisting of leukemic cells expressing CLEC12A, acute myeloid leukemia (AML), and subtypes thereof.
Item 17. The CAR-NK cell according to Item 7 or 13, the nucleic acid or the expression vector according to Item 11, the CAR-NK cell or CAR-T-cell according to Item 12 or the pharmaceutical composition according to Item 14 for use according to Item 15 or 16, wherein the prevention or treatment comprises a combination treatment with a hypomethylating agent, such as, for example, selected from azacitidine (AZA) or decitabine.
Item 18. A method for preventing or treating cancer, such as leukemia, or autoimmune diseases in a patient, comprising administering an effective amount of the CAR-NK cell according to Item 7 or 13, the nucleic acid or the expression vector according to Item 11, the CAR-NK cell or CAR-T-cell according to Item 12 or the pharmaceutical composition according to Item 14 to said patient.
Item 19. The method according to Item 18, wherein the prevention or treatment comprises a combination treatment with an effective amount of a hypomethylating agent, such as, for example, selected from azacitidine (AZA) or decitabine.
Item 20. The method according to Item 18 or 19, wherein the leukemia is selected from the group consisting of leukemic cells expressing CLEC12A, acute myeloid leukemia (AML), and subtypes thereof.

The invention will now be described further in the following examples with reference to the accompanying Figures and the accompanying sequence listing, nevertheless, without being limited thereto. For the purposes of the present invention, all references as cited are incorporated by reference in their entireties.

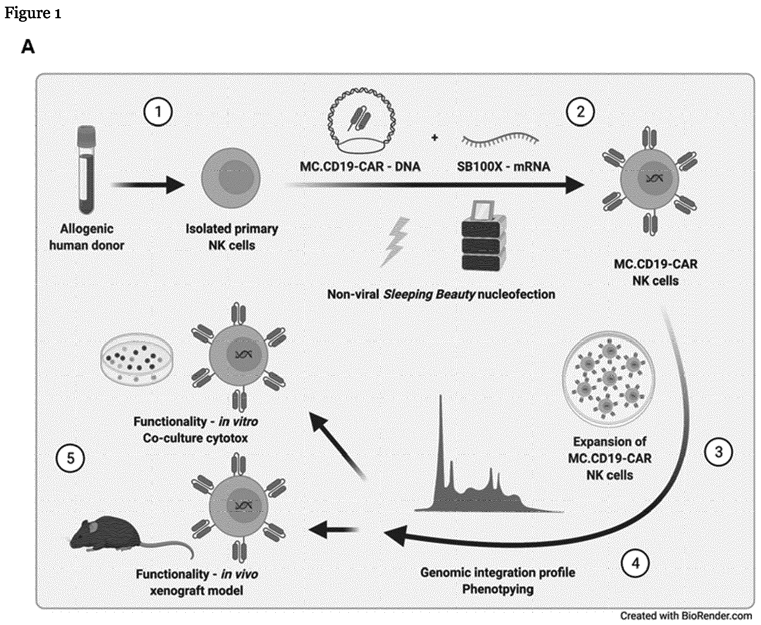

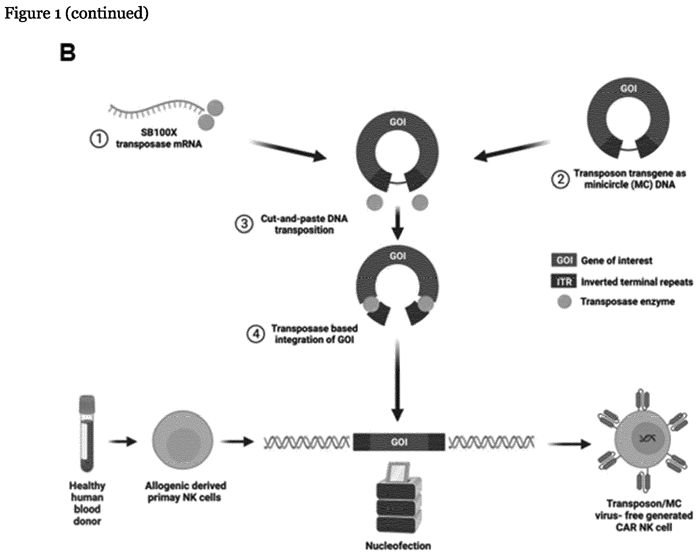

Figure 1 shows (A) Graphic abstract and workflow of the method to generate stable and non-viral genetically modified primary NK cells via Sleeping Beauty (SB) (SB100X) transposition and Minicircle DNA (MC) plasmids by virus-free nucleofection of primary NK cells. (B) Graphic representation of the workflow and schematic illustration of the Cut-and-paste DNA transposition followed by stable transgene integration using the SB transposon system. Primary NK cells were isolated from bone marrow aspirates of healthy patients and nucleofected with 1 µg of MC-gene-of-interest (GOI) (either Venus or CD19 or BCMA-CAR) together with 2.5 µg of active SB 100X transposase mRNA: 1-*4: SB* transposase mRNA molecules (circles) bind to the terminal inverted repeats (TIR) of the transposon cassette encoding the gene-of-interest (GOI, gray) and excises the transposable element from the donor plasmid. The excised transposon integrates to a new genomic location in the host genome.
Figure 2 shows (A) Frequency of stably expressing truncated EGFR (tEGFR) positive CD19-CAR NK cells between day 4 and day 23 post-nucleofection (n=4-10). (B) Integration frequencies of lentiviral (LV) and non-viral SB generated CAR NK cells in genomic safe harbors (GSH) of NK cells compared with random nucleotide frequency (computer-generated random positions in the genome map to GSHs). GSH are defined as regions of human chromosomes that fulfill the following five criteria shown of the *x* axis: not ultraconserved (UCE), >300 kb away from miRNA genes, >50 kb away from transcriptional start sites up- and downstream (TSS), >300 kb away from genes involved in cancer, >300 kb away from genes outside transcription units. Percentages of insertions fulfilling all five criteria. (C) Specific tumor cell lyses of CD19⁺ SUP-B 15 ALL cell line analyzed by flow cytometry after 4h of co-culture with NT and CD19-CAR NK cells at different effector to target (E:T) ratios (n=5-8). (D) Schematic representation of experimental layout for *in vivo* assessment of CD19-CAR NK cells in NALM6 (that express luciferase (luc⁺)) engrafted xenograft NSG mice. After inoculation of 0.5 × 10⁶ NALM6/luc leukemia cells, engraftment was confirmed in every mouse by BLI analysis. On day 3 1 × 10⁷ NT or CD19-CAR NK cells, pooled from three different donors, were intravenously injected, followed by daily subcutaneous administration of IL-2 and BLI imaging every 3-4 days. (E) Total flux analysis of BLI analyzes after leukemia engraftment (n=4) (Area under curve). (F) Representative BLI images of PBS injected, NT and CD19-CAR NK cells injected NALM-6/luc engrafted NSG mice 7, 11 and 14 days after leukemia engraftment are shown (n = 4 per group).

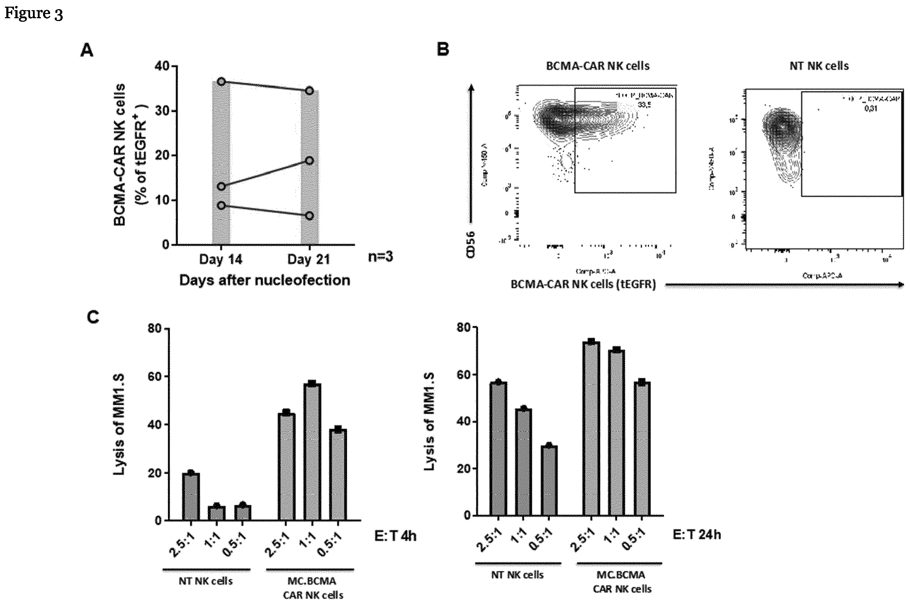

Figure 3 shows (A) Frequency of stably expressing truncated EGFR (tEGFR) positive BCMA-CAR NK cells between day 14 and day 21 post-nucleofection (n=3). (B) Representative FACS-plots of BCMA-CAR NK cells and non-transfected (NT) NK cells showing the BCMA-CAR/tEGFR expression in CD56⁺ cells (gated on viable and CD3⁻ /CD56⁺ cells). (C) Specific tumor cell lyses of BCMA⁺ MM1.S MM cell line analyzed by flow cytometry after 4h and 24h of co-culture with NT and BCMA-CAR NK cells at different effector to target (E:T) ratios.

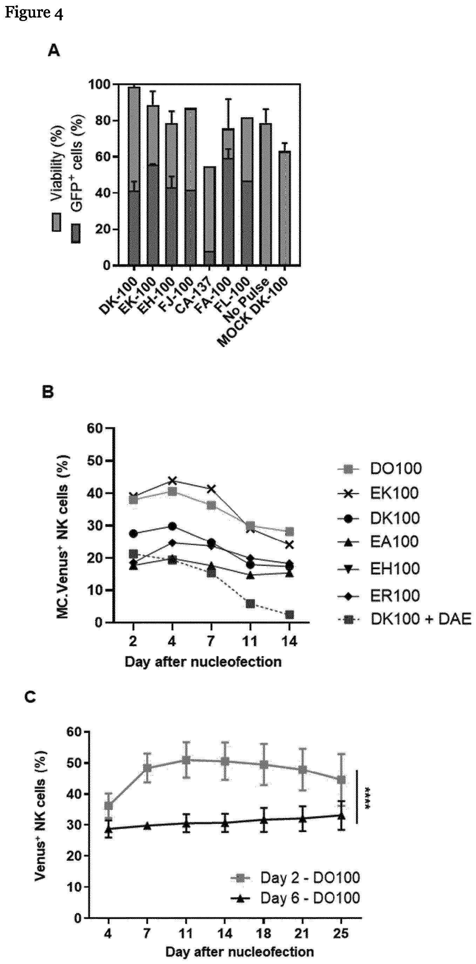

Figure 4 shows (A) Primary NK cells at day 6 of *in vitro* culture were nucleofected with pmaxGFP^{™} using different Lonza 4D Amaxa P3 nucleofection programs or with DK100 and without plasmid (MOCK). Frequency of pmaxGFP^{™} expressing NK cells and viability (FSC/SSC) was assessed 48 h post-nucleofection and compared to non-modified NK cell viability (No Pulse). (B) Primary NK cells at day 6 of *in vitro* culture were nucleofected with 1 µg MC Venus transposon (MC.T2-CAGGS-Venus) that carries a Venus reporter cassette together with 2.5 µg of a SNIM.RNA encoding the active SB100X transposase or with inactive SB100X (DAE) transposase using different Lonza 4D Amaxa P3 nucleofection programs. Program DK100 and DO100 resulted in strongest Venus expression over time. In the presence of inactive transposase SB100X(DAE), the initial levels of Venus expression gradually diminished during cell expansion being successively excluded from cell cultures with each cell division. In this case, Venus expression was undetectable by flow cytometry beyond 14 days post-delivery. (C) Primary NK cells at day 2 and 6 of *in vitro* culture were nucleofected using the nucleofection program DO100 and the frequency of Venus expressing cells was examined by flow cytometry 4-25 days post-nucleofection.
Figure 5 shows representative FACS-plots of NK cells after double modification by "one-step" nucleofection using SB/MC (Venus protein) and RNP-based CRISPR/Cas9 knockout (KO) nucleofection (EP). CRISPR/Cas9 is targeting *KLRC1* (encoding for NKG2A). (A) Non-treated NK cells (NT) stained for NKG2A after 14 days of cultivation with II,15 stimulation. (B) NK cells stained for NKG2A after NKG2A CRISPR/Cas9 KO 14 days post-nucleofection and expansion with IL15. (C) NK cells analyzed for Venus after SB100X-based MC-Venus nucleofection (upper row) and stained and analyzed for NKG2A expression (lower row). (D) NK cells analyzed for Venus after "one-step" double modification with SB 100X-based MC-Venus nucleofection and NKG2A CRISPR/Cas9 KO 14 days post-nucleofection and expansion with IL15. In the upper row double modified NK cells are analyzed for Venus expression at day 14 post-"one-step" nucleofection and showed 24 % of Venus expression and in the lower row same NK cell population is stained for NKG2A demonstrating 50% KO efficiency compared to NT NK cells at the same timepoint.
Figure 6 shows representative FACS-plots of NK cells after double modification by "one-step" nucleofection using SB/MC (BCMA-CAR) and RNP-based CRISPR/Cas9 knockout (KO) nucleofection (EP). CRISPR/Cas9 is targeting *KLRC1* (encoding for NKG2A). (A) NK cells stained for NKG2A after NKG2A CRISPR/Cas9 KO 21 days post-nucleofection and expansion with IL15 compared to non-treated NK cells (NT) at the same timepoint. (C) NK cells analyzed for tEGFR after SB 100X-based BCMA-CAR nucleofection and compared with NT NK cells. (D) NK cells analyzed for tEGFR after "one-step" double modification with SB 100X-based MC-BCMA-CAR nucleofection and NKG2A CRISPR/Cas9 KO 21 days post-nucleofection and expansion with IL15. Left is showing double modified NK cells analyzed for BCMA-expression (stained with anti-tEGFR antibody) at day 14 post-"one-step" nucleofection and the right FACS plot is showing NKG2A stained NK cells after double modification, demonstrating 50% KO efficiency compared to NT NK cells (see A) at the same timepoint. (D) Double modified BCMA/CAR-NKG2A KO NK cells stained for tEGFR and NGK2A demonstrating subpopulation of one-step modified NK cells after 21-day of cultivation with IL15.

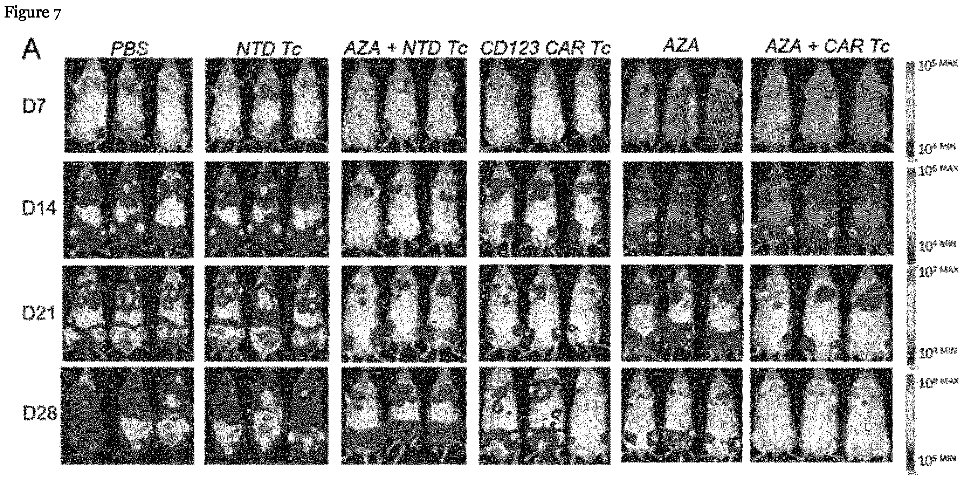

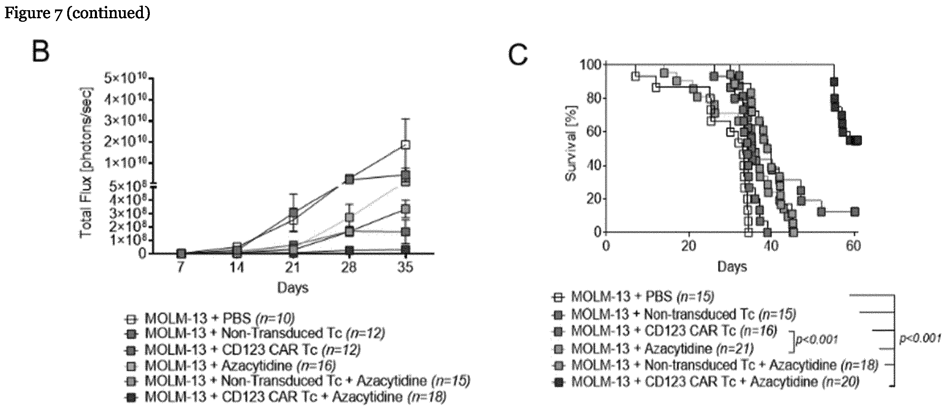

Figure 7 shows that azacytidine treatment enhances the anti-leukemic effect of CD 123-CAR-T cells. (A) Representative serial BLI depicting leukemia burden in MOLM-13 engrafted mice treated with PBS, NTD Tc, CD123-CAR-T cells, AZA only, AZA with NTD Tc, and AZA with CD123-CAR Tc. Data is represented colorimetrically with the scale bars indicating upper (max) and lower (min) BLI thresholds at each analysis time point. (B) BLI signals for each treatment group over time. Data were pooled from 2 independent experiments and at least 5 mice were alive at each time point. (C) Kaplan-Meier analysis of percentage survival for each treatment group. Data were pooled from 3 independent experiments. P-values were calculated using 2-sided Mantel-Cox test (log-rank).
Figure 8 shows CLEC12A-expression of AML cell lines. (A) Flow cytometry-based CLEC12A-expression analysis of different AML cell lines. (B) AZA-induced change of CLEC12A expression in OCI-AML3 cells. The fold change compares the CLE12A expression in the absence (day 0) versus presence (for 1, 4 or 8 days) of 1 µM azacytidine (AZA) in culture.
Figure 9 shows a schematic overview of CAR modules and the inventive optimized eCD8α transmembrane domain. (A) Overview of extracellular, transmembrane and intracellular CAR domains for the CLEC12A-specific CARs described in SEQ ID NO: 2 and SEQ ID NO: 3. (B) Shown are schematic representations of CLEC12A-CAR domains by the example of the described CLEC12A-CARs (SEQ ID NOs: 2 and 3) in a membrane-bound state. The membrane is illustrated as phospholipid bilayer. The size of each domain is only estimated and does not necessary reflect actual domain size. Notably, the left CAR utilizes a standard CD8α transmembrane domain while the optimized construct to the right (SEQ ID NOs: 2 and 3) comprise the enhanced CD8α transmembrane domain (eCD8α, SEQ ID NO: 4). Optimization of CD8α transmembrane domain was performed, by introducing 4 additional amino acids upstream and 2 additional amino acids downstream of the CD8α TM domain, towards enhanced membrane stability and signaling functionality. The following abbreviations are indicated: scFv = single chain variable Fragment; Myc-Tag = c-myc protein tag; CD8α hinge = hinge region of human CD8α chain; IgG4 hinge = hinge region of human IgG4 heavy chain; CD8α TM = transmembrane domain of human CD8α chain; eCD8α TM = enhanced transmembrane domain of human CD8α chain ; 4-1BB = costimulatory domain of human 4-1BB (CD137); CD3ζ= Human CD3ζ chain signaling domain.
Figure 10 shows the CAR transduction efficacy *in vitro* on NK cells following lentiviral transduction. Surface expression level of a CLEC12A-targeting CAR on primary T cells at day 6 and on primary NK cells at day 11 after lentiviral transduction are indicated.
Figure 11 shows an in vivo xenograft model of OCI-AML2. (A) Scheme of the *in vivo* evaluation of AML treatment with CLEC12A-CAR-NK cells (10 × 10⁶ i.v.) in combination with s.c. IL-2 support in a Luc+-OCI-AML2 xenograft NSG-SGM3 mouse model. (B) BLI-analysis reveals reduced leukemic burden in mice treated with CLEC12A-CAR-NK cells. The 4-1BB-based CAR construct achieved superior results compared to non-transduced NK cells and NK cells engineered with a CD28-based CAR construct.
Figure 12 shows a schematic representation of the CAR modules for the CAR construct CLEC12A.IgG4h.eCD8αTM.BBz (see also SEQ ID NO: 3). Implemented downstream of the CAR are a truncated EGFR (tEGFR) which functions as a safety switch and a cytokine, such as soluble human IL-15 (sIL-15) for enhanced *in vivo* persistence of the NK cells.

SEQ ID NO: 1 shows the amino acid sequence for the human CLEC12A protein.

SEQ ID NO: 2 shows the amino acid sequence for the CAR construct CLEC12A.CD8αh.eCD8αTM.BBz.

SEQ ID NO: 3 shows the amino acid sequence for the CAR construct CLEC12A.IgG4h.eCD8αTM.BBz.

SEQ ID NO: 4 shows the optimized transmembrane amino acid sequence for the eCD8α TM according to the present invention: FACDIYIWAPLAGTCGVLLLSLVITLY

### Examples

### Material and Methods

### NK cell isolation and cultivation

Primary NK cells are isolated and generally cultivated feeder-cell free with IL15 (PeproTech) as previously described (Bexte T, et al. CRISPR-Cas9 based gene editing of the immune checkpoint NKG2A enhances NK cell mediated cytotoxicity against multiple myeloma. Oncoimmunology. 2022 May 31;11(1):2081415. doi: 10.1080/2162402X.2022.2081415. PMID: 35694192; PMCID: PMC9176243). Briefly, primary NK cells are isolated from anonymous donor buffy coats using Ficoll density gradient centrifugation (Biocoll, Biochrom) and immunomagnetic negative NK cell selection (EasySep Human NK cell Enrichment Kit, Stem Cell Technology).

### Preparation of plasmids, MC DNA, and SB100X SNIM mRNA transposase

To establish and optimize the nucleofection protocol for primary NK cells, the pmaxGFP plasmid (4D Nucleofector, Lonza) and a MC-expressing Venus fluorescent protein are used in combination with the SB100X mRNA transposase. MC Venus and MCs encoding CLEC12A-CAR transposons are generated similar to what was described for CD19 (Müller et al., 2019; Monjezi et al., 2017). SB100X mRNA transposase was kindly provided by Ethris GmbH (Planegg) as an SNIM RNA.

### Generation and nucleofection of primary SB-generated CAR NK cells

NK cells are isolated from the peripheral blood of healthy donors and nucleofected with either pmaxGFP alone or SB transposons vectorized as MCs, which encode either a Venus fluorescent protein or a CAR (e.g. targeting BCMA, CD19 or CLEC12A) in combination with a hyperactive SB100X transposase. The transposon MC vectors carrying BCMAR-CAR are flanked by ITRs and are provided in the form of SNIM mRNA SB100X. Per sample, 1 × 10⁶ NK cells are nucleofected with 2.5 µg of SB transposase and 1 µg MC DNA using the 4D Nucleofector (Lonza), P3 Primary Cell 4D-Nucleofector X Kit S and L, and the DO100 nucleofection program. After nucleofection, the cells are transferred to cultivation medium.

In a preferred protocol, for a pretreatment CD56neg selected NK cells were isolated and cultivated for 2 days with the addition of 10 ng/ml IL15. Then, in preparation for nucleofection, the cells were centrifuged, and washed with pre-warmed medium. After a first centrifugation, the supernatant was discarded with a pipette, followed by a short spinfection (about 10 sec). Then, again, the supernatant was carefully removed, followed by the addition of a nucleofection buffer (e.g. P3 buffer). As the Lonza 4D program (Pulse), program DO100 was used (alternatively, DK100, EK100, EH100, FJ100, CA137, FA100, FL100, CM137, DK-125, EA100, or ER100 were also successfully applied). After the pulse, pre-warmed medium was added, followed by a recovery period for 15-20min in an incubator. The nucleofected NK cells are then transferred into a pre-warmed cultivation well and IL15 (10ng/mL) was added.

### Phenotyping and flow cytometry

To evaluate NK purity and CAR expression efficiency during *ex-vivo* expansion, CAR - and NT NK cells are stained with the following antibodies: CD56-BV786, CD3-BUV395 (all BD), and SA-PE (BD) or MycTag (clone 9B11, Cell Signaling Technology) and 7AAD (BD).

For phenotyping following antibodies are used as recently described (Bexte T, et al. CRISPR-Cas9 based gene editing of the immune checkpoint NKG2A enhances NK cell mediated cytotoxicity against multiple myeloma. Oncoimmunology. 2022 May 31;11(1):2081415. doi: 10.1080/2162402X.2022.2081415. PMID: 35694192; PMCID: PMC9176243): CD45-BUV395 (BD), CD16-BUV395 (BD), CD56-BV786 (BD), CD3-PerCP (Biolegend), CD19-PerCP (Biolegend), CD14 - PerCP (Miltenyi), CD16-PE-CF594 (BD), 7AAD (BD), CD56-BV421 (BD), CD69-BV605 (BD), NKG2C-AF488 (R&D), NKp46-BV421 (BD), KIR2D-FITC (Miltenyi), NKp30-BV605 (BD), NKp44-BV711 (BD), CD62L-BB515 (BD), CXCR4-BV421 (BD Horizon), CX3CR1-BV605 (BD), CCR7-BV711 (BD), FasL-PE (Biolegend), CD16-PE-CF594 (BD). Gating strategy for phenotyping surface markers: Single cells, FSC/SSC, CD3-CD19-CD14-7AAD^{neg} cells, CD45^{pos}, CD56^{pos} cells using a BD FACSCelesta flow cytometer (BD Biosciences). Data are analyzed using FlowJo (FlowJo LLC).

### In vivo xenograft mouse studies with SB-generated CD19-CAR NK cells

The therapeutic effect of CAR NK cells was examined in a mouse xenograft model of human ALL. Appearance, behavior, and weight of animals was monitored every 2-3 days and tumor load is analyzed. The appearance, behavior, and weight of the animals was monitored every 2-3 days and progress of leukemia is analyzed. Once the termination criteria had been reached, the mice were sacrificed.

### In vivo xenograft mouse studies with OCI-AML2 and CLEC12A CAR-NK cells

The therapeutic effect of CAR NK cells was examined in a mouse xenograft model of human AML. Appearance, behavior, and weight of animals was monitored every 2-3 days and tumor load is analyzed.

### Statistical Analysis

Statistical analysis was performed using GraphPad Prism version 6 and 9 (La Jolla, CA, USA). If not mentioned otherwise, differences between experimental groups was assessed using Student's t-test, under the assumption of normal distribution of NK function in healthy donors. All *p*-values reported are two-sided and are considered significant at 0.05. Only data from experiments with three or more donors (n ≥ 3) were considered for statistical analysis.

### Summary of experimental approach

For the experiments in the context of the present invention, the herein-provided engineered NK cell products, preferably generated by use of non-viral transposon-based technology (alternatively by viral transduction) including CLEC12A-CAR-NK, NKG2A-KO-NK, CLEC12A-CAR/NKG2A-KO-NK cells were used.

The SB integration site data were evaluated based on transposition events into genes (% of total insertions that occurred in genes), transcriptional status of the targeted genes in NK cells, the location of the insertion with respect to gene structure (5' regulatory regions, exons, introns), insertion into repetitive DNA, the base composition at the target regions, and association with a panel of histone markers representing transcriptionally active and inactive chromatin.

Provided was thus a preclinical risk assessment of the potential genotoxic risks by SB- and CRISPR-technology with regard to future optimization of non-viral NK cell engineering and employment of the CAR NK cell product in clinical trials.

### Preclinical in vivo evaluation of the combination therapy concept with AZA and

### CLEC12A-CAR/KO-NK cells

Here, the inventors took advantage of the state-of-the art *in vitro* and *in vivo* models to study CAR-NK cell mediated AML elimination available to the applicants. The aim was to test and optimize efficacy of combined demethylating therapy and CAR/KO-NK cells for treatment of AML. A second aim is to understand toxicity of the combination against normal HSCs. Thirdly leukemia immune escape from the CAR-NK cells by secretion of immunosuppressive metabolites and the contribution of the bone marrow microvasculature to the homing and functionality of CAR-immune cells was assessed.

Preclinical experiments of the CLEC12A-CAR/KO-NK cell preparations in combination with AZA-pretreatment were carried out to evaluate anti-leukemic cytotoxicity and explore toxicities as well as immune evasion in preclinical *in vivo* mouse models. The inventors further investigated the cellular and molecular mechanisms that regulate interactions between CLEC12A-CAR/KO-NK and leukemia cells. The inventors studied the specific role of the bone marrow microvasculature for CLEC12A-CAR/KO-NK efficacy.

For doing so, the inventors determined the ideal combination of CLEC12A-CAR/KO-NK cells and AZA with respect to timing and cytotoxic activity. To achieve this goal, the inventors tested CLEC12A-targeting CAR/NKG2A-KO-NK cells compared to CLEC12A-CAR-NK, NKG2A-KO-NK or unmodified NK cells from the same donor *ex vivo* and *in vivo.* In a first set of experiments, the inventors employed (i) 2D/3D bone marrow niche models using patient-derived materials and addition of immune cells (e.g. myeloid cells, primary and engineered immune cells), and (ii) Xenograft models such as MOLM13 or OCI-AML2 in NSG-SGM3 mice, as well as (iii) PDX models in humanized non-conditioned NSG mice that maintain the integrity of the microenvironment for assessment of CAR-immune cell cytotoxic function against patient-derived AML blasts *in vivo.*

## Claims

1. A method for producing a CAR-NK cell expressing an antigen-targeting chimeric antigen receptor (CAR) or a functional alternatively spliced transcript variant thereof, comprising the steps of
a) providing primary NK cells,
b) pretreating the primary NK cells for about 2 days in a suitable medium comprising about 10 ng/ml IL15,
c) nucleofecting the cells with i) at least one Minicircle DNA encoding a transposon cassette element comprising a nucleic acid encoding the CAR or the functional alternatively spliced transcript variant thereof, and ii) a nucleic acid encoding at least one Sleeping Beauty (SB 100X) transposase using a suitable nucleofection program, such as, for example, DO100, and
d) expanding the nucleofected cells in a suitable medium comprising about 10 ng/ml IL15.

2. The method according to claim 1, wherein the transformation efficiency of the NK cells is at least 30%, preferably at least 40%, and more preferably at least 50%, and wherein preferably a stable integration is achieved.

3. The method according to claim 1 or 2, wherein the nucleic acid is an mRNA molecule, such as an SNIM mRNA.

4. The method according to any one of claims 1 to 3, wherein the transposable element furthermore encodes for one or more cytokines, chemokines, receptors, suicide genes and/or transfection markers.

5. The method according to any one of claims 1 to 4, wherein the CAR binds to an antigen selected from BCMA, CD19 and CLEC12A, and wherein the CAR NK cells are preferably genetically modified.

6. The method according to any one of claims 1 to 5, further comprising the step of rendering non-functional at least one immune checkpoint and/or regulatory receptor protein, such as, for example NKG2A, PD-1, and/or KIRS, wherein preferably the immune checkpoint receptor inactivation comprises a genetic deletion, a CRISPR/Cas introduced mutation, RNA interference (RNAi) and/or the recombinant expression of an immune checkpoint receptor inhibiting peptide.

7. A recombinant CAR-NK cell, produced according to the method according to any one of claims 1 to 6.

8. A CAR-construct, comprising a modified CD8α transmembrane domain comprising between 3 to 5, preferably 4 additional amino acids at the N-terminus and between 1 to 3, preferably 2 additional amino acids at the C-terminus of the CD8α or CD28 transmembrane (TM) domain, preferably a CAR-construct comprising the amino acid sequence according to SEQ ID No. 4, more preferably wherein the CAR binds to a tumor-associated antigen, such as an antigen selected from BCMA, CD19 and CLEC12A.

9. A nucleic acid encoding for the CAR-construct according to claim 8 or an expression vector comprising said nucleic acid.

10. A recombinant CAR-NK cell or CAR-T-cell, comprising the CAR-construct according to claim 8 or expressing the nucleic acid or expression vector according to claim 9.

11. A recombinant CAR-NK cell, expressing a CAR binding to the antigen CLEC12A or a functional alternatively spliced transcript variant thereof, wherein at least one immune checkpoint receptor protein, such as, for example NKG2A, PD-1, and/or KIRS, is inactivated, wherein preferably the immune checkpoint receptor inactivation comprises a genetic deletion, a CRISPR/Cas introduced mutation, RNA interference (RNAi) and/or the recombinant expression of an immune checkpoint receptor inhibiting peptide.

12. A pharmaceutical composition, comprising the CAR-NK cell according to claim 7 or 11, the nucleic acid or the expression vector according to claim 9, or the CAR-NK cell or CAR-T-cell according to claim 10, together with at least one pharmaceutically acceptable diluent or carrier.

13. The CAR-NK cell according to claim 7 or 11, the nucleic acid or the expression vector according to claim 9, the CAR-NK cell or CAR-T-cell according to claim 10 or the pharmaceutical composition according to claim 12 for use in medicine, in particular for use in the prevention or treatment of cancer, such as leukemia, or autoimmune diseases in a patient.

14. The CAR-NK cell according to claim 7 or 11, the nucleic acid or the expression vector according to claim 9, the CAR-NK cell or CAR-T-cell according to claim 10 or the pharmaceutical composition according to claim 12 for use according to claim 13, wherein the leukemia is selected from the group consisting of leukemic cells expressing CLEC12A, acute myeloid leukemia (AML), and subtypes thereof.

15. The CAR-NK cell according to claim 7 or 11, the nucleic acid or the expression vector according to claim 9, the CAR-NK cell or CAR-T-cell according to claim 10 or the pharmaceutical composition according to claim 12 for use according to claim 13 or 14, wherein the prevention or treatment comprises a combination treatment with a hypomethylating agent, such as, for example, selected from azacitidine (AZA) or decitabine.
